# EUROPEAN PATENT SPECIFICATION

(11) **EP 4 210 687 B1**
(45) Date of publication and mention of the grant of the patent: **27.05.2026**
(21) Application number: 21790525.6
(22) Date of filing: 10.09.2021
(51) Int. Cl.: A61K 31/165, A61P 17/02

(54) **TRPV1 AGONISTS IN THE PREVENTION OF DIABETIC ULCERS**
TRPV1 AGONISTEN IN DER PRÄVENTION VON DIABETISCHEN GESCHWÜREN
AGONISTES TRPV1 DANS LA PRÉVENTION D'ULCÈRES DIABÉTIQUES

(30) Priority: 10.09.2020 GB 202014256
(43) Date of publication of application: 19.07.2023
(73) Proprietor: Imperial College Innovations Limited, London SW7 2AZ (GB)
(72) Inventor: ANAND, Praveen, London Greater London SW72AZ (GB)
(74) Representative: Abel & Imray LLP
(86) International application number: PCT/GB2021/052349
(87) International publication number: WO 2022/053820

(56) References cited:
- FRYKBERG ROBERT G ET AL: "Management of Diabetic Foot Ulcers: A Review", FEDERAL PRACTITIONER, 1 February 2016 (2016-02-01), pages 16 - 23, XP055875587, Retrieved from the Internet <URL:https://www.ncbi.nlm.nih.gov/pmc/articles/PMC6368931/pdf/fp-33-02-16.pdf> [retrieved on 20211222]
- ANAND PRAVEEN ET AL: "Rational treatment of chemotherapy-induced peripheral neuropathy with capsaicin 8% patch: from pain relief towards disease modification", JOURNAL OF PAIN RESEARCH, vol. Volume 12, 3 July 2019 (2019-07-03), pages 2039 - 2052, XP055804118, Retrieved from the Internet <URL:https://www.dovepress.com/getfile.php?fileID=50915> DOI: 10.2147/JPR.S213912
- FORST T. ET AL: "The influence of local capsaicin treatment on small nerve fibre function and neurovascular control in symptomatic diabetic neuropathy", ACTA DIABETOL, vol. 39, 1 January 2002 (2002-01-01), pages 1 - 6, XP055858350, Retrieved from the Internet <URL:https://link.springer.com/content/pdf/10.1007/s005920200005.pdf>
- LYNN R WEBSTER ET AL: "Efficacy, safety, and tolerability of NGX-4010, capsaicin 8% patch, in an open-label study of patients with peripheral neuropathic pain", DIABETES RESEARCH AND CLINICAL PRACTICE, AMSTERDAM, NL, vol. 93, no. 2, 11 April 2011 (2011-04-11), pages 187 - 197, XP028260756, ISSN: 0168-8227, [retrieved on 20110414], DOI: 10.1016/J.DIABRES.2011.04.010
- SIMPSON DAVID M ET AL: "Capsaicin 8% Patch in Painful Diabetic Peripheral Neuropathy: A Randomized, Double-Blind, Placebo-Controlled Study", JOURNAL OF PAIN, SAUNDERS, PHILADELPHIA, PA, US, vol. 18, no. 1, 13 October 2016 (2016-10-13), pages 42 - 53, XP029862614, ISSN: 1526-5900, DOI: 10.1016/J.JPAIN.2016.09.008

## Description

### Field of the Invention

The present invention concerns diabetic peripheral neuropathy. More particularly, this invention relates to capsaicin, dihydrocapsaicin, nordihydrocapsaicin, homocapsaicin, homodihydrocapsaicin, capsiate, nonivamide or a TRPV1 agonist for use in the prevention of diabetic ulcers in a patient suffering from diabetic peripheral neuropathy, when the capsaicin, dihydrocapsaicin, nordihydrocapsaicin, homocapsaicin, homodihydrocapsaicin, capsiate, nonivamide or TRPV1 agonist is administered topically to one or more areas of the skin. The invention also concerns related medical uses.

### Background of the Invention

Peripheral neuropathy is a general term used to describe a condition wherein the peripheral nerves (nerves that lie beyond the brain and spinal cord) are damaged. Peripheral neuropathy can be induced, for example, by metabolic disorders such as diabetes mellitus, traumatic injuries, immune conditions or infections, or exposure to toxins. Peripheral neuropathy can be painful (for example, it may involve painful shooting sensations, and/or burning pain) or non-painful (for example, it may involve sensations of numbness, loss of balance, or non-painful spontaneous tingling).

Diabetic peripheral neuropathy (DPN) refers to peripheral neuropathy (whether painful or non-painful) induced by diabetes mellitus. The underlying mechanisms are diverse (Anand et al., Nat. Med. 1996, 2:703-707; Anand et al., Br. J. Anaesth. 2011, 107:490-502; Gylfadottir SS et al., J Diabetes Investig. 2019, 10: 1148-57). In this connection, progressive loss of nerve fibres has been found to occur in diabetic patients, in particular those with longstanding diabetic peripheral neuropathy (Narayanaswamy et al., J. Clin. Neurosci. 2012, 19:1490-1496).

Current treatments for neuropathic pain in patients experiencing painful DPN have limited efficacy and significant side-effects (Finnerup et al., Lancet Neurol. 2015, 14:162-73; Anand et al., Pain Manag. 2019, 9:521-533). These include tricyclic antidepressants (e.g. amitriptyline), serotonin noradrenaline reuptake inhibitors (e.g. duloxetine and venlafaxine), pregabalin and gabapentin. Other treatments are lidocaine patches, topical capsaicin including the capsaicin 8% patch (Qutenza^{®}), and tapentadol. At present, there are no disease-modifying therapies licensed for the treatment of DPN (Javed et al., Diabet. Med. 2020, 37:573-79).

Capsaicin is an agonist of TRPV1 (Transient Receptor Potential Cation Channel Subfamily V Member 1), a non-selective cation channel that functions as an integrator of noxious chemical and physical stimuli. Topical capsaicin formulations can be used to manage pain. For example, low-concentration creams are currently available for daily skin application over the course of several weeks. Capsaicin 8% Patch (also known as the Capsaicin 179 mg Cutaneous Patch, or by the proprietary name Qutenza^{®} and available from Grunenthal Ltd) is considered an effective, safe and well-tolerated topical treatment for the relief of neuropathic pain. Capsaicin 8% Patch is at present licensed in the EU and other territories for the treatment of peripheral neuropathic pain. Without wishing to be bound by theory, it is thought that capsaicin alleviates pain by overstimulating skin nociceptors, leading to the skin nociceptors being temporarily incapacitated, i.e., 'defunctionalised' (Anand and Bley, Br. J. Anaesth. 2011, 107:490-502; Anand et al., Pain Manag. 2019, 9:521-533). It has been found that pain relief may persist in DPN patients for months after a single 30-minute application of Capsaicin 8% Patch (Martini et al., J Pain Res. 2012, 5:51-59). Progressive pain relief has also been reported in DPN patients treated repeatedly with Capsaicin 8% Patch, at ≥ 8-week intervals, over the course of a 52-week study (Vinik et al., BMC Neurol. 2016, 16(1):251).

Previous studies to determine the effect of various topical capsaicin formulations on the skin have demonstrated that degeneration of cutaneous sensory nerve terminals occurs (as part of 'defunctionalisation') followed by cutaneous sensory nerve terminal regeneration. The time-course of nerve fibre degeneration and then regeneration over weeks following high dose topical capsaicin in skin biopsies have been reported in a human volunteer study (Ragé et al., Clin Neurophysiol. 2010, 121(8):1256-1266), and after capsaicin 8% patch application (Kennedy et al., J Pain, 2010; 11(6):579-587). Topical treatments with capsaicin cream, in clinical experimental studies, resulted in slower intra-epidermal nerve regeneration (following degeneration) in patients with diabetes mellitus, compared to healthy control subjects, particularly in those with DPN (Polydefkis et al., Brain 2004, 127:1606-1615), and type 2 diabetes mellitus (Khoshnoodi et al., Ann Clin Transl Neurol. 2019, 6:2088-2096).

Anand et al., J Pain Res. 2019, 2:2039-2052 assesses the therapeutic potential of capsaicin 8% patch in patients with painful CIPN, and its mechanism of action.

Forst et al., Acta Diabetol., 2002, 39 :1-6 investigates the impact of topical capsaicin application on small nerve fibre function and neurovascular control.

Webster et al., Diabetes Res. And Clin. Pract., 2011, 93, 2:187-197 assesses the efficacy, safety, and tolerability of a capsaicin 8% patch in patients with peripheral neuropathic pain.

Simpson et al., J. Pain, 2016, 18, 1 :42-53 concerns a 12-week study evaluating the efficacy and safety of capsaicin 8% patch versus placebo patch in painful diabetic peripheral neuropathy (PDPN).

Diabetics, particularly those with peripheral neuropathy, are susceptible to the development of diabetic ulcers. Diabetes can lead to neuropathy and foot ulcers, impede the normal steps of the wound healing process, prolong ulcers and lead to major discomfort and morbidity. It will be appreciated that diabetic ulcers occurring on patients' limbs, for example foot ulcers, can necessitate amputation of the limb in question. Diabetic ulcers, for example diabetic foot ulcers, present a major global burden to patients and to their carers and physicians.

### Summary of the Invention

As defined in claim 1, the present invention provides capsaicin, dihydrocapsaicin, nordihydrocapsaicin, homocapsaicin, homodihydrocapsaicin, capsiate, nonivamide or a TRPV1 agonist for use in the prevention of diabetic ulcers in a patient suffering from diabetic peripheral neuropathy, wherein the capsaicin, dihydrocapsaicin, nordihydrocapsaicin, homocapsaicin, homodihydrocapsaicin, capsiate, nonivamide or TRPV1 agonist is administered topically to one or more areas of the skin. Further optional features are defined in the dependent claims.

### Description of the Drawings

Embodiments of the present invention will now be described, by way of example only, with reference to the accompanying schematic drawings.

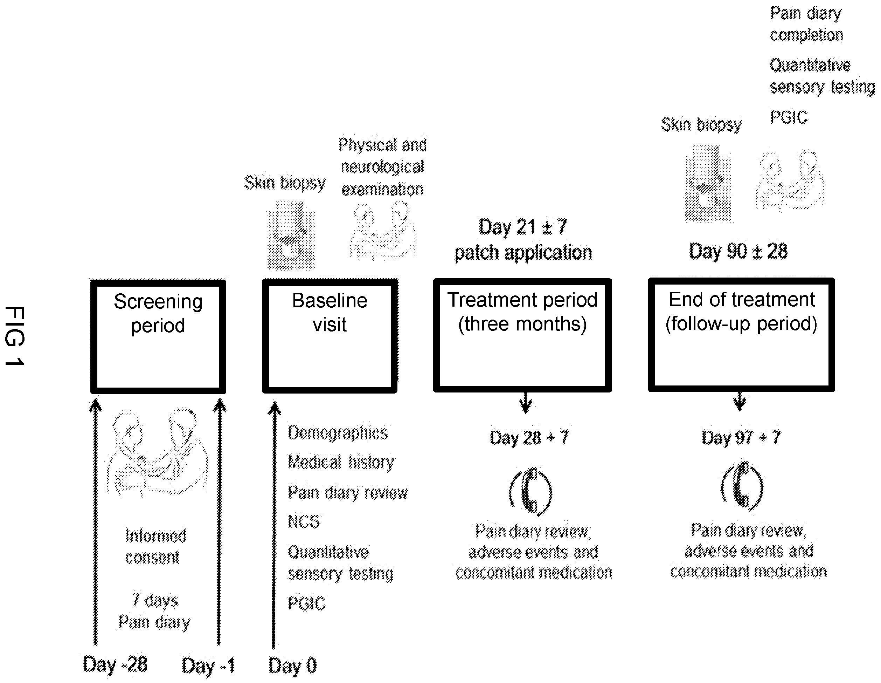

**Figures 1 to 18** show findings of a study conducted with subjects having painful diabetic peripheral neuropathy.
**Figure 1** shows a study flow diagram illustrating the sequence of assessment and treatment for patients included in the Capsaicin 8% Patch treatment and Standard of Care (Q+SOC) group of the study. Patients had the option to have assessments and Capsaicin 8% Patch treatment repeated 3 monthly for 12 months. Abbreviations: NCS: Nerve conduction study, PGIC: Patient Global Impression of Change.

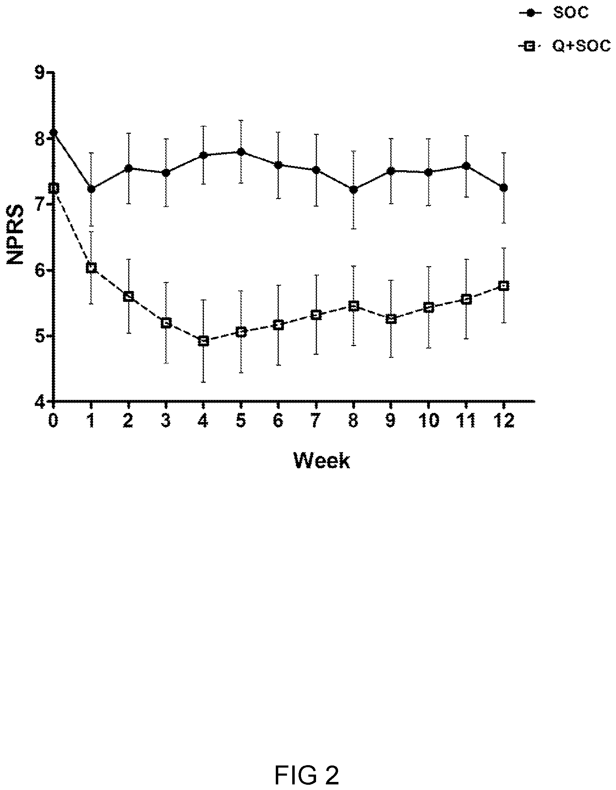

**Figure 2** shows the time-course graph of spontaneous pain scores over 12 weeks in the two study groups, Capsaicin 8% Patch Qutenza (Q) plus Standard of Care group (Q+SOC; n=25), and Standard of Care group (SOC; n=12). Statistically significant difference was detected between Q+SOC and SOC groups for NPRS scores 3 months after the baseline visit (***p<0.0001). Separate assessment of pain scores at each week time point showed a significant difference in NPRS scores at week 3 (*p<0.05), week 4 (**p<0.01), week 5 (*p<0.05), week 6 (*p<0.05) and week 9 (*p<0.05; two-way ANOVA analysis).

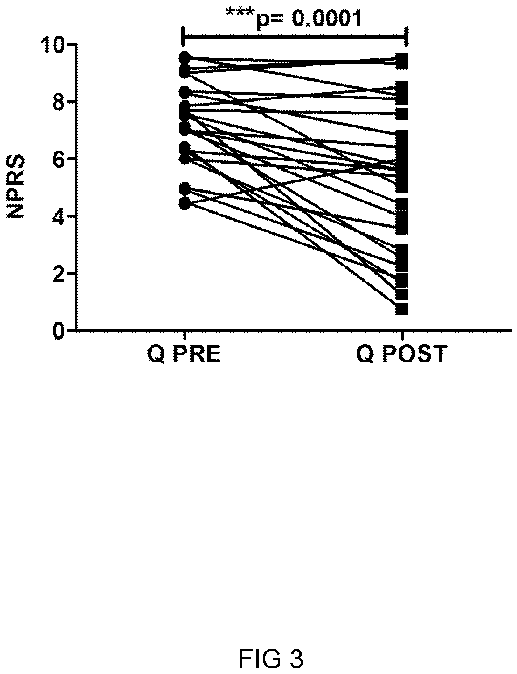

**Figure 3** shows pain scores of patients having painful diabetic peripheral neuropathy (Q+SOC, n=25) treated with Capsaicin 8% Patch Qutenza (Q), at pre-treatment visit (Q PRE) and 3 months after treatment (Q POST), on Numerical Pain Rating Scale (NPRS). Statistically significant difference at 3 months after treatment was detected (***p= 0.0001; n= 25; paired t- test).

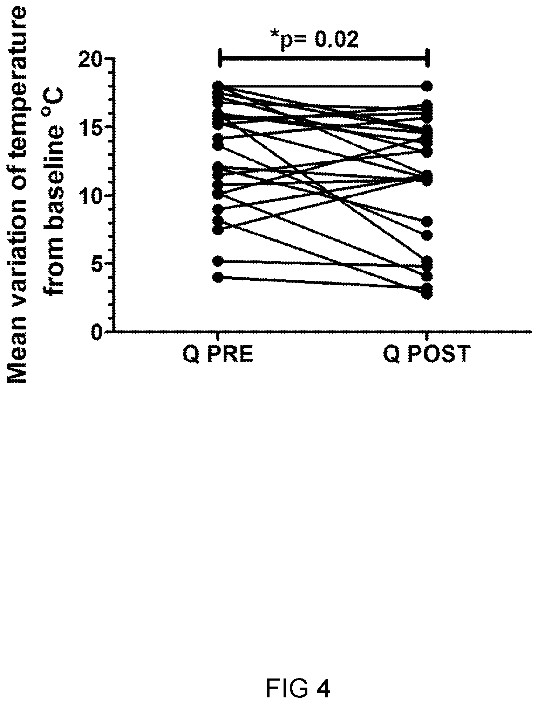

**Figure 4** shows the thermal thresholds for warm perception (changes from baseline at 32°C), in Q+SOC group treated with Capsaicin 8% Patch Qutenza (Q), at pre-treatment visit (Q PRE) and visit 3 months after treatment (Q POST). Statistically significant difference (improvement) 3 months after the treatment was detected (*p= 0.02; paired t-test).

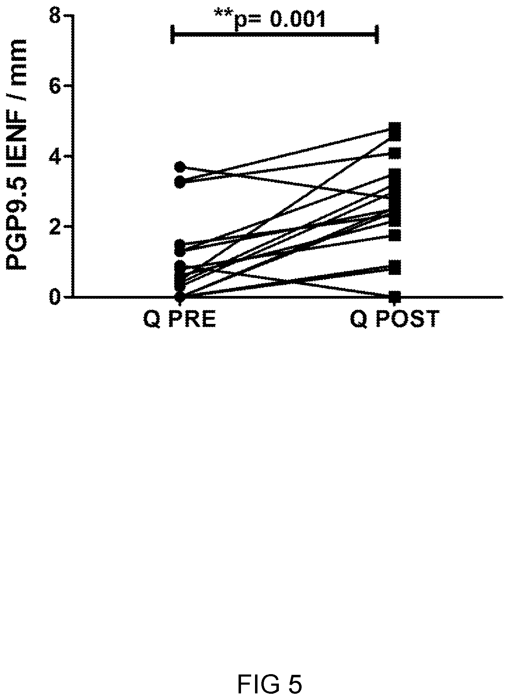

**Figure 5** shows immunohistochemistry in skin biopsies for intra-epidermal PGP9.5 nerve fibre density (IENFs/mm; 15 µm sections) in Q+SOC group treated with Capsaicin 8% Patch Qutenza (Q), at pre-treatment visit (Q PRE) and visit 3 months after treatment (Q POST). Statistically significant increase of IENFs PGP9.5 nerve fibres at 3 months after Capsaicin 8% Patch application was detected (**p=0.001; paired t-test; n=25).

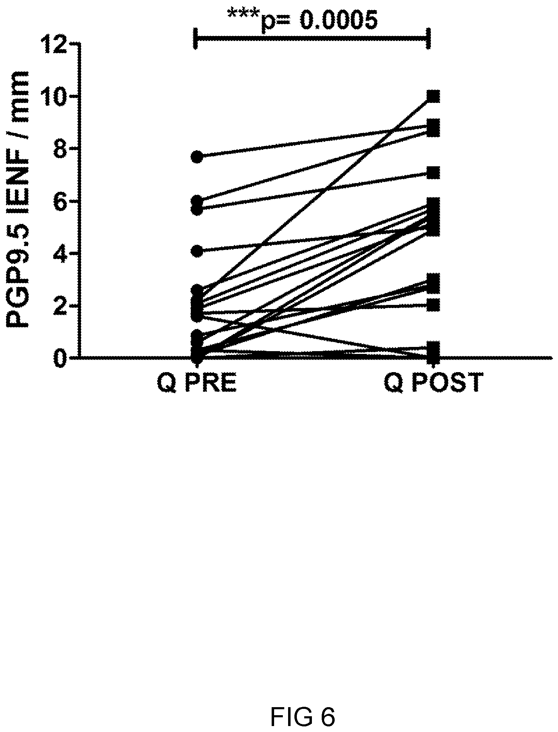

**Figure 6** shows immunohistochemistry in skin biopsies for intra-epidermal PGP9.5 nerve fibres (IENFs/mm; 50 µm sections) in Q+SOC group treated with Capsaicin 8% Patch Qutenza (Q), at pre-treatment visit (Q PRE) and visit 3 months after treatment (Q POST). Statistically significant increase of IENFs PGP9.5 nerve fibres at 3 months after Capsaicin 8% Patch application was detected (***p=0.0005; paired t-test; n=25).

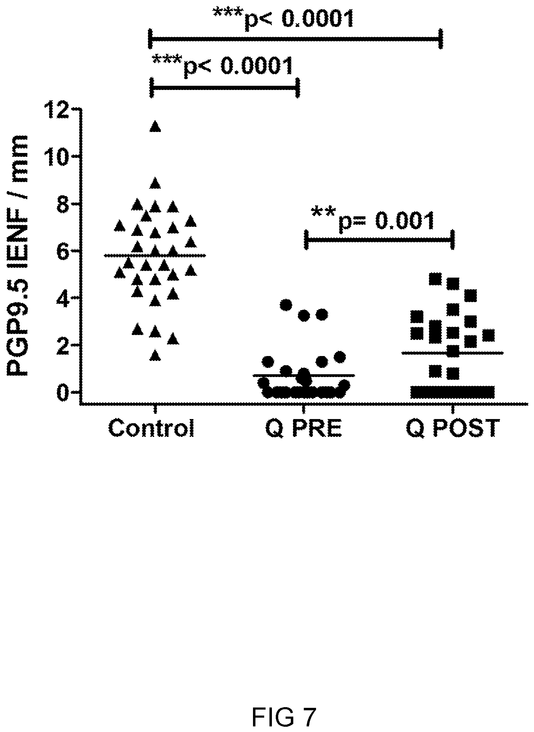

**Figure 7** shows immunohistochemistry in skin biopsies for intra-epidermal PGP9.5 nerve fibres (IENFs/mm; 15 µm sections) in Q+SOC group treated with Capsaicin 8% Patch Qutenza (Q), at pre-treatment visit (Q PRE) and visit 3 months after treatment (Q POST). Statistically significant difference between control group and treated group before (***p<0.0001; Mann-Whitney test) and after Capsaicin 8% Patch application (***p<0.0001; Mann-Whitney test) was detected.

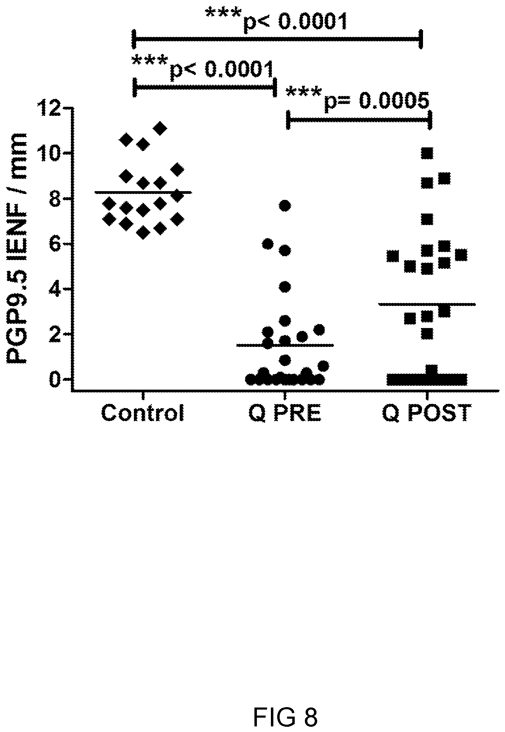

**Figure 8** shows immunohistochemistry in skin biopsies for intra-epidermal PGP9.5 nerve fibres (IENFs/mm; 50 µm sections) in Q+SOC group treated with Capsaicin 8% Patch Qutenza (Q), at pre-treatment visit (Q PRE) and visit 3 months after treatment (Q POST). Statistically significant difference between control group and treated group before (***p<0.0001; Mann-Whitney test) and after Capsaicin 8% Patch application (***p<0.0001; Mann-Whitney test) was detected.

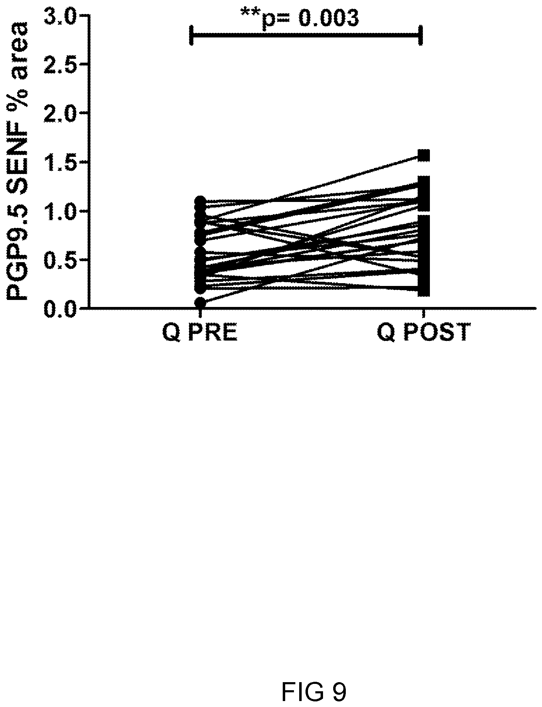

**Figure 9** shows immunohistochemistry in skin biopsies for sub-epidermal PGP9.5 nerve fibres (SENFs; % Area; 15 µm sections) in Q+SOC group treated with Capsaicin 8% Patch Qutenza (Q), at pre-treatment visit (Q PRE) and visit 3 months after treatment (Q POST). Statistically significant increase of SENFs PGP9.5 nerve fibres at 3 months after Capsaicin 8% Patch application (**p=0.003; paired t-test; n=25) was detected.

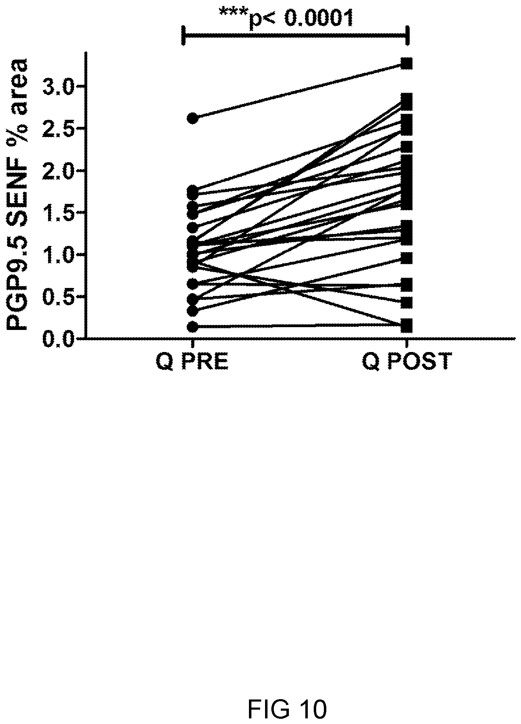

**Figure 10** shows immunohistochemistry in skin biopsies for sub-epidermal PGP9.5 nerve fibres (SENFs; % Area; 50 µm sections) in Q+SOC group treated with Capsaicin 8% Patch Qutenza (Q), at pre-treatment visit (Q PRE) and visit 3 months after treatment (Q POST). Statistically significant increase of IENFs PGP9.5 nerve fibres at 3 months after Capsaicin 8% Patch application (***p<0.0001; paired t-test; n=25) was detected.

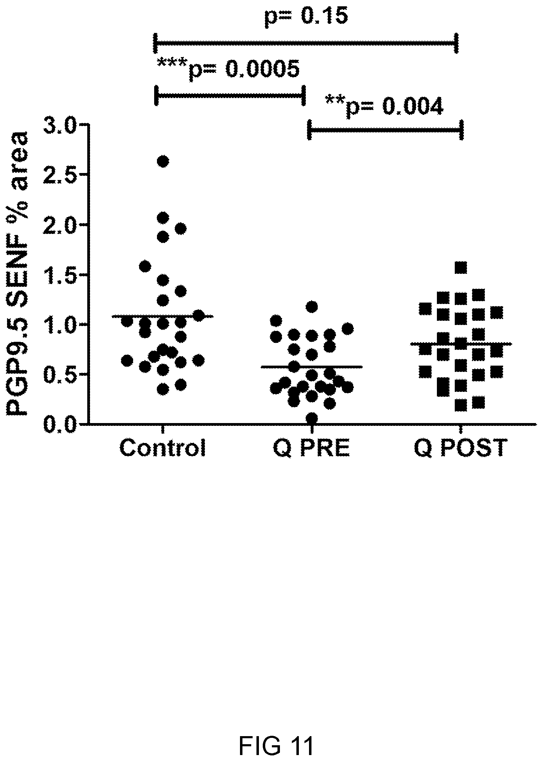

**Figure 11** shows immunohistochemistry in skin biopsies for sub-epidermal PGP9.5 nerve fibres (SENFs; % Area; 15 µm sections) in Q+SOC group treated with Capsaicin 8% Patch Qutenza (Q), at pre-treatment visit (Q PRE) and visit 3 months after treatment (Q POST). Statistically significant difference between control group and treated group before (***p=0.0005; Mann-Whitney test) but not after Capsaicin 8% Patch application (p=0.15; Mann-Whitney test) was detected.

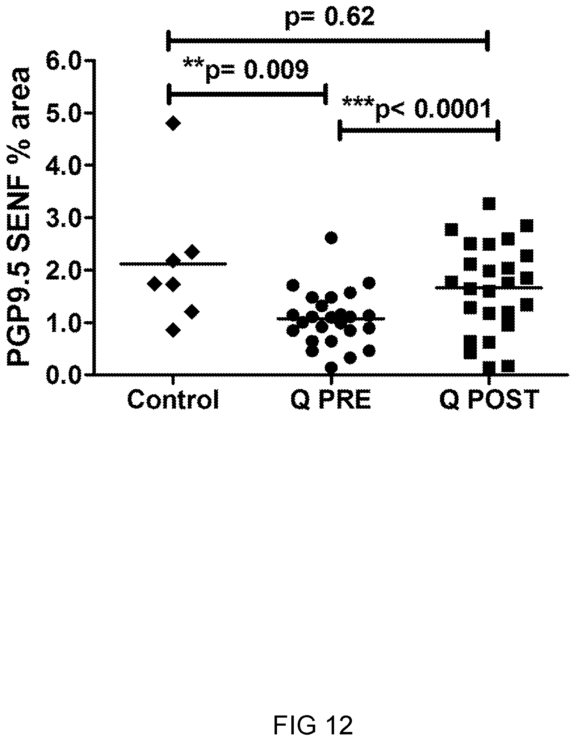

**Figure 12** shows immunohistochemistry in skin biopsies for sub-epidermal PGP9.5 nerve fibres (SENFs; % Area; 50 µm sections) in Q+SOC group treated with Capsaicin 8% Patch Qutenza (Q), at pre-treatment visit (Q PRE) and visit 3 months after treatment (Q POST). Statistically significant difference between control group and treated group before (**p=0.009; Mann-Whitney test) but not after Capsaicin 8% Patch application (p=0.62; Mann-Whitney test) was detected.

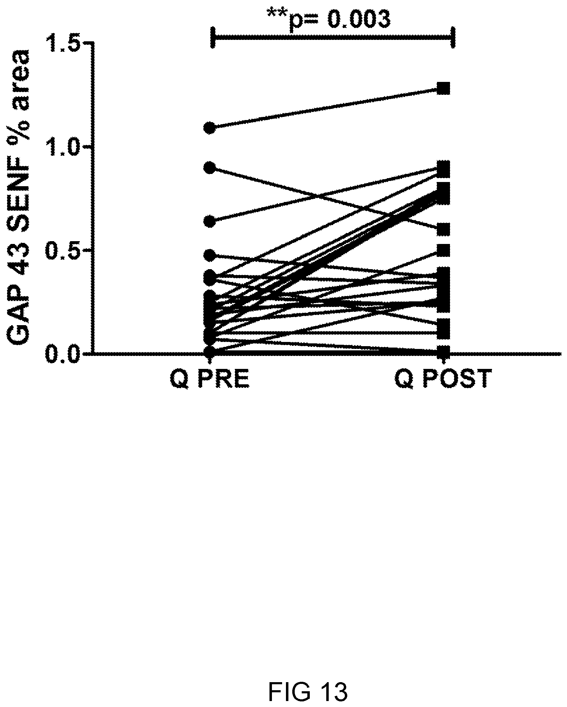

**Figure 13** shows immunohistochemistry in skin biopsies for sub-epidermal GAP43 nerve fibres (SENFs; % Area) in Q+SOC group treated with Capsaicin 8% Patch Qutenza (Q), at pre-treatment visit (Q PRE) and visit 3 months after treatment (Q POST). Statistically significant increase of SENFs GAP43 nerve fibres at 3 months after Capsaicin 8% Patch application (**p=0.003; paired t-test; n=25) was detected.

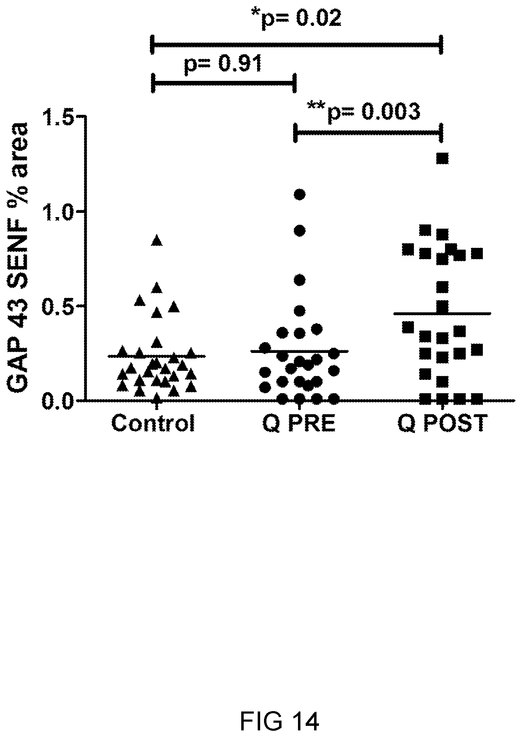

**Figure 14** shows immunohistochemistry in skin biopsies for sub-epidermal GAP43 nerve fibres (SENFs/mm) in Q+SOC group treated with Capsaicin 8% Patch Qutenza (Q), at pre-treatment visit (Q PRE) and visit 3 months after treatment (Q POST). Statistically no significant difference was detected between control group and treated group before treatment (p=0.91; Mann-Whitney test), but statistically significant difference between control group and treated group after Capsaicin 8% Patch application (*p=0.02; Mann-Whitney t test) was detected.

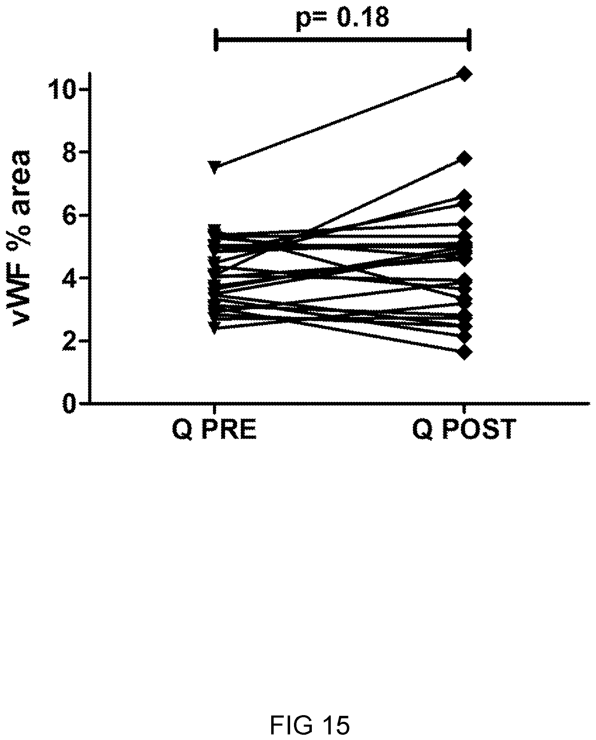

**Figure 15** shows immunohistochemistry in skin biopsies for vWF (% Area) in Q+SOC group at pre-treatment visit (Q PRE) and visit 3 months after treatment (Q POST). No statistically significant difference of vWF % area at 3 months after Capsaicin 8% Patch application (p=0.18; paired t-test; n=25) was detected.

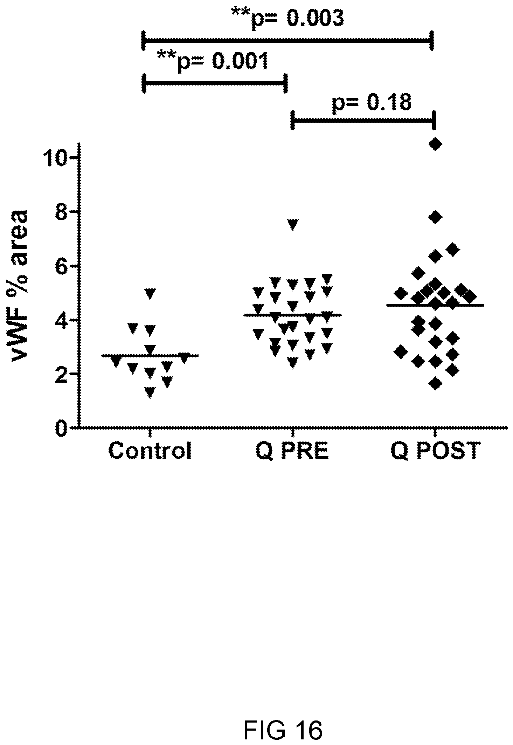

**Figure 16** shows immunohistochemistry in skin biopsies for vWF (% Area) in Q+SOC group treated with Capsaicin 8% Patch Qutenza (Q), at pre-treatment visit (Q PRE) and visit 3 months after treatment (Q POST). Statistically significant difference between control group and treated group before (**p=0.001; Mann-Whitney test) and after Capsaicin 8% Patch application (**p=0.004; Mann-Whitney test) was detected.

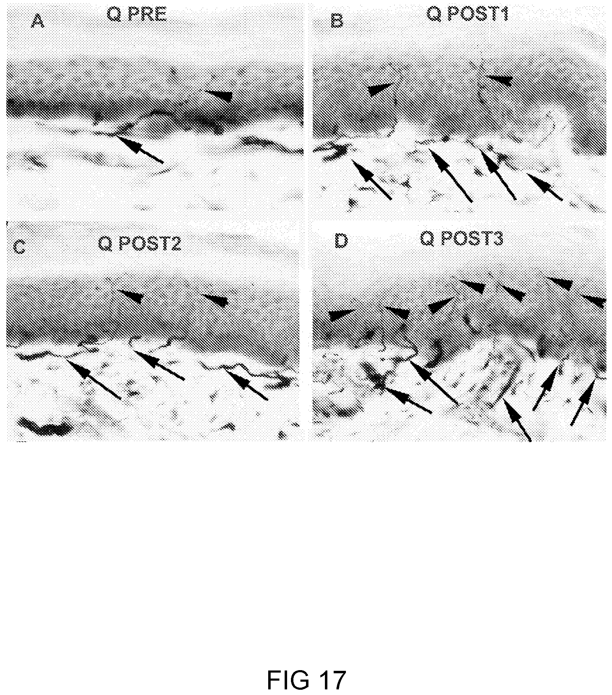

**Figure 17** shows staining for PGP9.5 before and after application of Qutenza in 50µm thick sections (patient in Case Report). A: Few intra-epidermal nerve fibres with abnormal trajectory (here oblique) were observed before capsaicin application (arrowheads, Q PRE). B, C and D: Examples from biopsies collected 3. 6 and 9 months after single Qutenza application respectively (Q POST 1, 2, 3). Note the restored abundance of both IENF and SENF, and the vertical trajectory of IENF Q POST, as observed in control skin. Magnification x20.

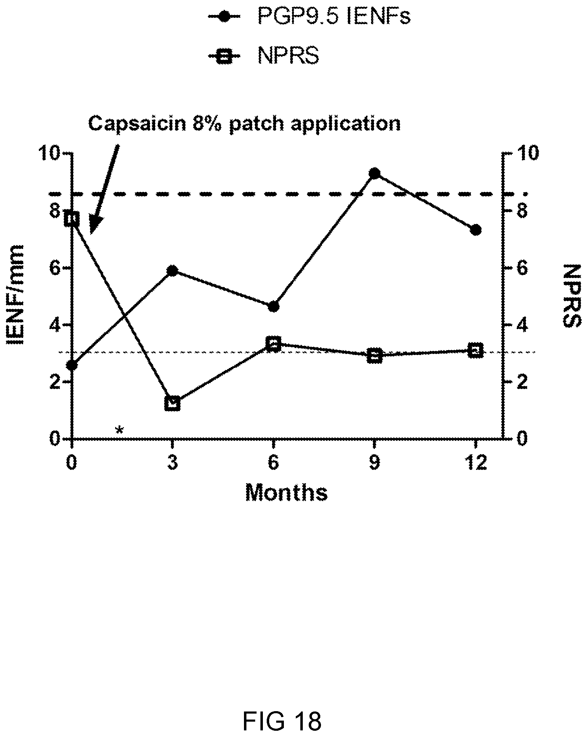

**Figure 18** shows the time course for NPRS scores (spontaneous pain) and PGP9.5 IENFs in 50µm sections at 3, 6, 9 and 12 months following a single Qutenza application (patient in Case Report). While pain relief was maintained, PGP9.5 immunostaining revealed a significant progressive increase in density of intra-epidermal nerve fibre density (IENFs), 3 months and onwards (thick horizontal dashed line is median value for age and gender in a published normative data study, values below the thin dotted horizontal line are considered likely to be abnormal (0.05 quantile).

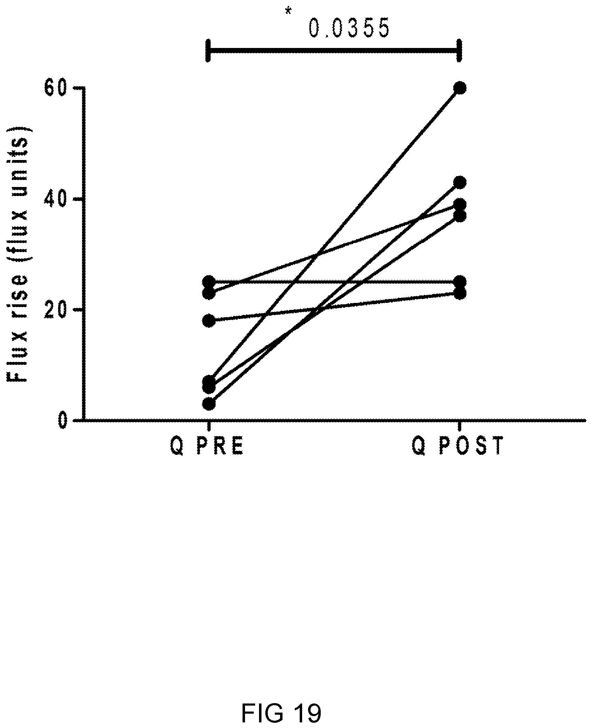

**Figures 19 to 24** show findings of an extension of the study, to subjects having non-painful diabetic peripheral neuropathy.
**Figure 19** shows axon-reflex vasodilation at baseline (Q PRE) and 3 months follow-up visit (Q POST) in patients with non-painful diabetic peripheral neuropathy treated with Capsaicin 8% patch, Qutenza (Q). Axon-reflex vasodilation in skin was significantly increased between baseline and 3 months follow-up visit (n=6, p=0.0355, paired t-test). The test stimulus used was the Capsaicin 8% patch (2 cm x 2 cm) applied on the dorsum foot skin for 10 minutes, and the increase of capillary flux (peak minus baseline) measured using a laser-Doppler (Perimed, Stockholm). Previous studies of capsaicin-induced axon-reflex vasodilation showed that baseline values in the calf of similar patients with diabetic neuropathy were approximately 40% lower than in control subjects (Anand et al., Nat. Med. 1996, 2:703-707), suggesting that the increased flux after 3 months in this study was within the normal range.

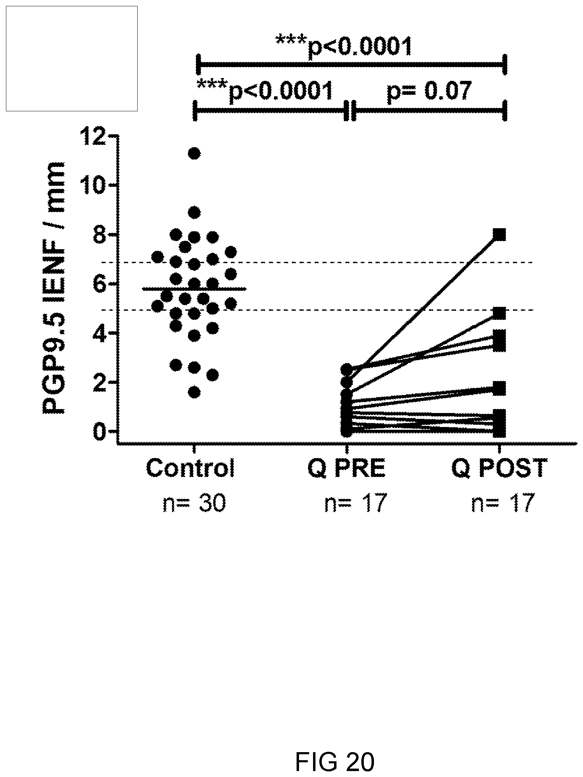

**Figure 20** shows, for patients with non-painful diabetic peripheral neuropathy, intra-epidermal PGP9.5 nerve fibres (IENFs/mm) at baseline (Q PRE), and non-significant increase at 3 months after treatment (Q POST) with Capsaicin 8% patch Qutenza (Q) (paired t-test). There is a statistically significant difference between control group and baseline (Q PRE), and after treatment (Q POST) (Mann-Whitney test).

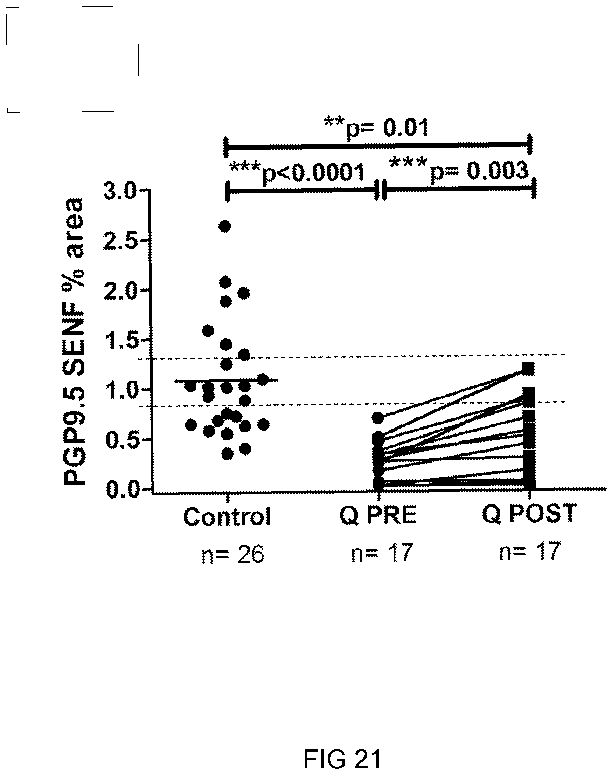

**Figure 21** shows, for patients with non-painful diabetic peripheral neuropathy, sub-epidermal PGP9.5 nerve fibres (SENFs; % Area) at baseline (Q PRE) and 3 months after treatment (Q POST) with Capsaicin 8% patch Qutenza (Q). There is a statistically significant increase after Capsaicin 8% patch application (paired t-test). There is a statistically significant difference between control and treated group before (Q PRE) but less significant after (Q POST) Capsaicin 8% patch application (Mann-Whitney test).

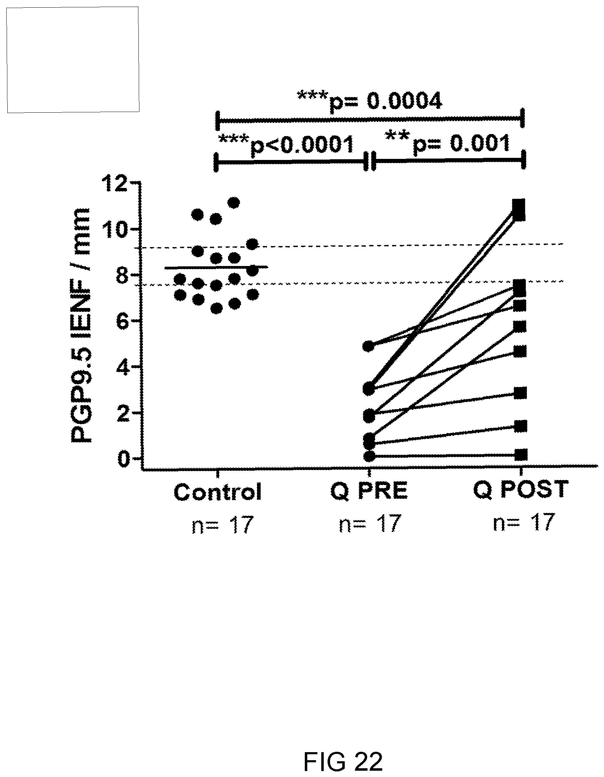

**Figure 22** shows, for patients with non-painful diabetic peripheral neuropathy, intra-epidermal PGP9.5 nerve fibres (IENFs/mm) at baseline (Q PRE), and a significant increase at 3 months after treatment (Q POST) with Capsaicin 8% patch Qutenza (Q) (paired t-test). There is a statistically significant difference between control group and baseline (Q PRE), but less significant after treatment (Q POST) (Mann-Whitney test).

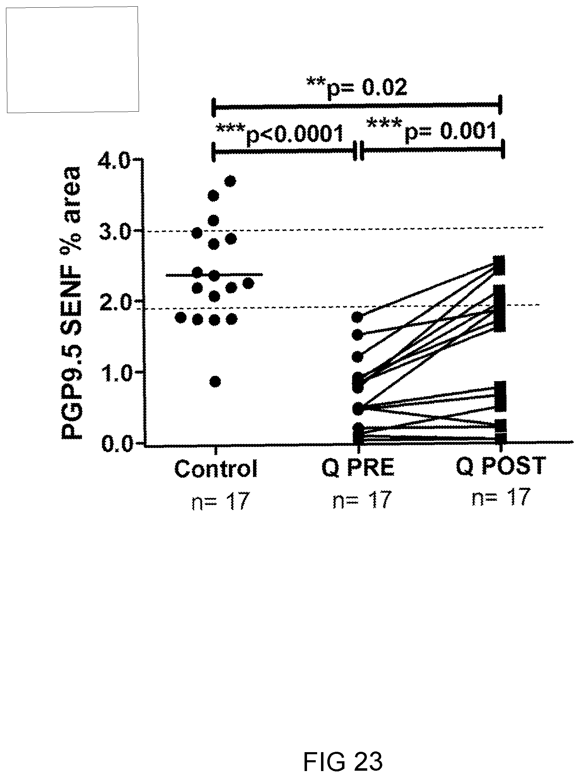

**Figure 23** shows, for patients with non-painful diabetic peripheral neuropathy, sub-epidermal PGP9.5 nerve fibres (SENFs; % Area) at baseline (Q PRE) and 3 months after treatment (Q POST) with Capsaicin 8% patch Qutenza (Q). Statistically significant increase after Capsaicin 8% patch application was detected (paired t-test). Statistically significant difference between control and treated group before (Q PRE) but less after (Q POST) Capsaicin 8% patch application was detected (Mann-Whitney test).

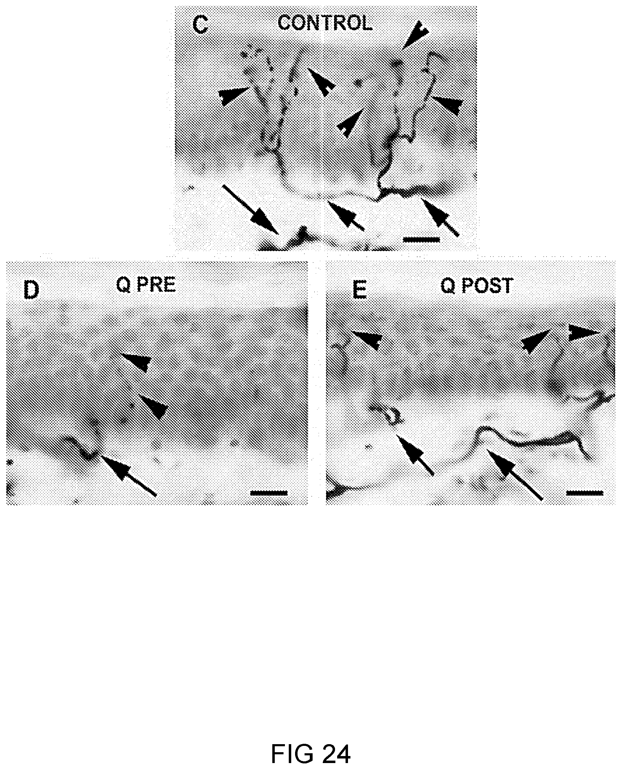

**Figure 24** shows: (C) control skin biopsy section, from a healthy human volunteer (intra-epidermal nerve fibre marked with arrowhead, and sub-epidermal nerve fibre with arrow); (D) skin biopsy section from a subject with painless DPN pre-treatment (Q PRE) - few intra-epidermal nerve fibres and sub-epidermal nerve fibres were observed before Capsaicin 8% patch Qutenza (Q) application; and (E) skin biopsy section from same subject with painless DPN as above post-treatment (Q POST); 3 months after Capsaicin 8% patch application, the abundance of both the IENFs and SENFs appeared restored. (Scale bar 50 µm). Magnification x 40.

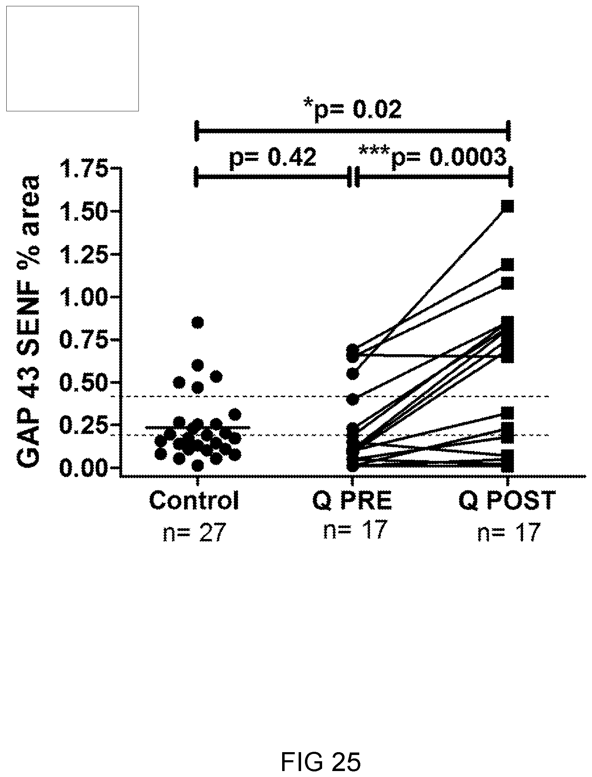

**Figure 25** shows, for patients with non-painful diabetic peripheral neuropathy, sub-epidermal GAP43 nerve fibres (SENFs; % Area) at pre-treatment visit (Q PRE) and visit 3 months after treatment (Q POST) with Capsaicin 8% patch Qutenza (Q). There is a statistically significant increase of SENFs at 3 months after Capsaicin 8% patch application (paired t-test). There is a statistically non-significant difference between control group and treated group before treatment (Q PRE), but statistically significant difference between control group and treated group after Capsaicin 8% patch application (Q POST) (Mann-Whitney test).

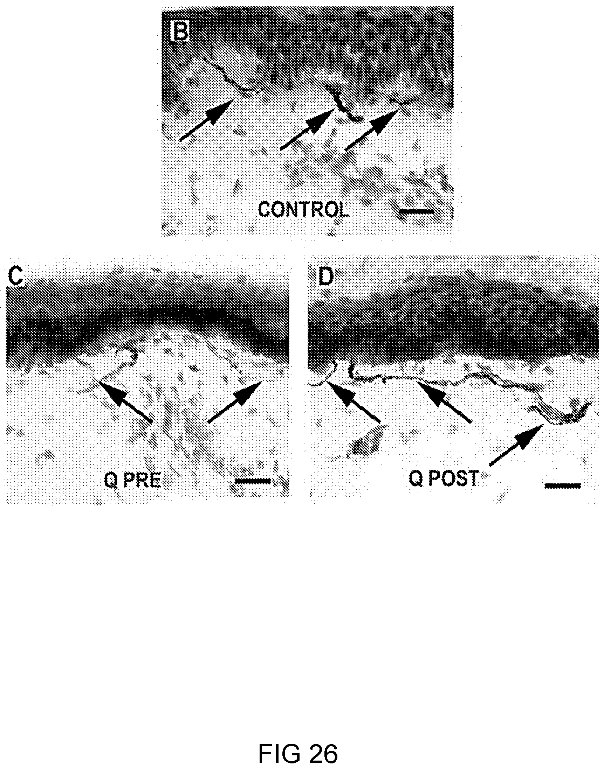

**Figure 26** shows: (B) control skin biopsy section, from a healthy human volunteer (sub-epidermal nerve fibre with arrow); (C) skin biopsy section from a participant with painless DPN pre-treatment (Q PRE); fewer sub-epidermal nerve fibres than control were observed before Capsaicin 8% patch Qutenza (Q) application; and (D) skin biopsy section from same participant with painless DPN as above post-treatment (Q POST); 3 months after Capsaicin 8% patch application, the abundance and length of SENFs appeared increased. (Scale bar 50 µm). Magnification x 40.

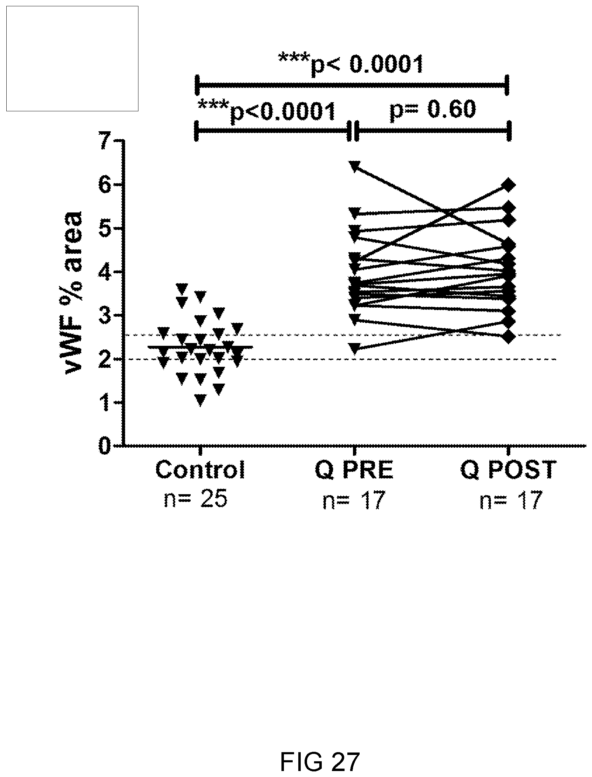

**Figure 27** shows, for patients with non-painful diabetic peripheral neuropathy, vWF vessels (% Area) at pre-treatment visit (Q PRE) and visit 3 months after treatment (Q POST) with Capsaicin 8% patch Qutenza (Q). There is a statistically non-significant difference at 3 months after Capsaicin 8% patch application (paired t-test). There is a statistically significant difference between the control group and treated group before treatment (Q PRE) and after Capsaicin 8% patch application (Q POST) (Mann-Whitney test).

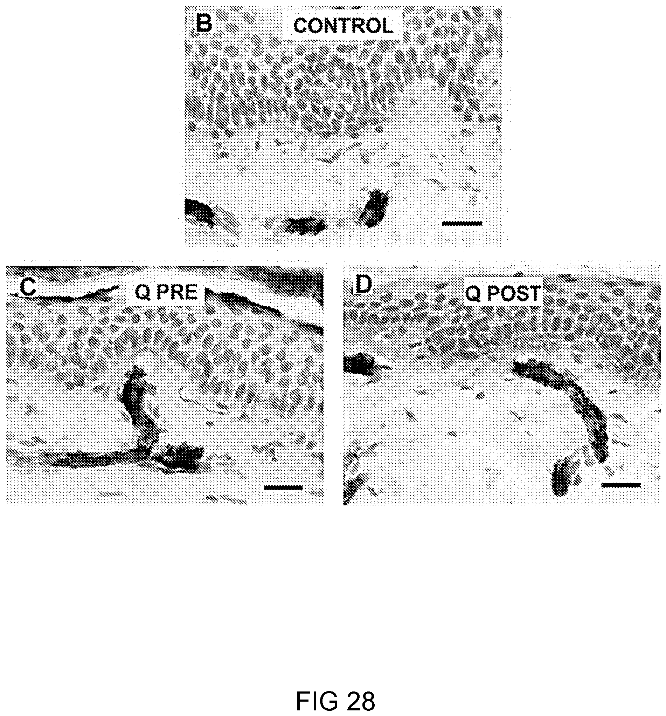

**Figure 28** shows: (B) control skin biopsy from a healthy human volunteer (vessels stained in black); (C) skin biopsy from subject with painless DPN pre-treatment (Q PRE) - note increase in vessels; (D) skin biopsy section from same subject with painless DPN, post-treatment (Q POST), at 3 months after Capsaicin 8% patch application, blood vessels remain unchanged. (Scale bar 50 µm). Magnification x 40.

### Definitions

### Diabetes mellitus

Diabetes mellitus, commonly referred to simply as diabetes, is a term used to describe a metabolic disorder of multiple aetiology, which is characterized by chronic hyperglycaemia with disturbances of carbohydrate, fat and protein metabolism resulting from defects in insulin secretion, insulin action, or both. Diabetes mellitus can result in the long-term damage, dysfunction and failure of various organs.

Typically, symptoms of diabetes can include frequent urination, increased thirst, and increased appetite. Acute complications of diabetes, particularly if left untreated, can include diabetic ketoacidosis, hyperosmolar hyperglycaemic state, and death. Serious long-term complications of diabetes tend to include cardiovascular disease, stroke, chronic kidney disease, diabetic ulcers such as foot ulcers, damage to the nerves, damage to the eyes and cognitive impairment.

Diabetes results from diminished insulin production by the pancreas, or a failure by cells to properly respond to insulin. Diabetes can be sub-divided into three main types. Type 1 diabetes results from a failure of the pancreas to produce sufficient insulin, due to a loss of pancreatic beta cells, typically caused by an autoimmune response. Type 2 diabetes initially involves insulin resistance (wherein cells fail to properly respond to insulin) and as the disease progresses may additionally involve a lack of insulin production. Common causes of type 2 diabetes include a combination of excessive body weight and insufficient exercise. The third main type of diabetes is gestational diabetes, which occurs in pregnant women who develop high blood sugar levels; this can occur in women not having a previous history of diabetes.

Certain preferred embodiments of the invention relate to type 1 diabetes, for example poorly controlled or uncontrolled type 1 diabetes. Certain preferred embodiments of the invention relate to type 2 diabetes, for example poorly controlled or uncontrolled type 2 diabetes. Certain preferred embodiments of the invention relate to gestational diabetes. Certain preferred embodiments of the invention relate to type 1 or type 2 diabetes, for example poorly controlled or uncontrolled type 1 or type 2 diabetes.

Certain preferred embodiments of the invention relate to diabetes that is poorly controlled. Certain preferred embodiments of the invention relate to diabetes that is uncontrolled. For example, patients suffering from poorly controlled or uncontrolled diabetes may have an average blood sugar level over time (for example, over the course of 6 months, or over the course of 12 months) that is significantly higher (for example, 1 mmol/L or even higher) than the average blood sugar level over time of a healthy subject not suffering from diabetes. Patients suffering from poorly controlled or uncontrolled diabetes may experience frequent infections. Patients suffering from poorly controlled or uncontrolled diabetes may develop diabetic ulcers, for example diabetic foot ulcers. Certain preferred embodiments of the invention concern diabetic patients who have already suffered from one or more diabetic ulcers, for example one or more diabetic foot ulcers. Certain preferred embodiments of the invention concern diabetic patients who are currently suffering from one or more diabetic ulcers, for example one or more diabetic foot ulcers. Certain preferred embodiments of the invention concern diabetic patients liable to suffer from foot ulcers. A liability to suffer from diabetic foot ulcers can be indicated by the patient having poorly controlled or uncontrolled diabetes. A liability to suffer from diabetic foot ulcers can be indicated, for example, by the patient having suffered from one or more diabetic foot ulcers previously. A liability to suffer from diabetic foot ulcers on one foot can be indicated, for example, by the patient suffering from one or more diabetic foot ulcers on their other foot. A liability to suffer from diabetic foot ulcers can be indicated, for example, by the patient having a peripheral neuropathy, for example a peripheral neuropathy on the foot. Such a neuropathy may be painful or non-painful, and may be chronic or non-chronic. In some embodiments, a liability to suffer from diabetic foot ulcers can be indicated, for example, by the patient having a chronic peripheral neuropathy, for example a chronic peripheral neuropathy of the foot.

### Peripheral neuropathy

Peripheral neuropathy refers to a condition wherein the peripheral nerves (nerves that lie beyond the brain and spinal cord) are damaged. Exemplary causes of peripheral neuropathy include metabolic disorders, immune conditions or infections, traumatic injuries, and exposure to toxins. Peripheral neuropathy can be chronic (chronic peripheral neuropathy refers to long-lasting peripheral neuropathy, persisting for more than a few months, for example more than 3 months, or more than 6 months).

Peripheral neuropathy affecting sensory nerves can be painful. Painful peripheral neuropathy refers to peripheral neuropathy resulting in painful symptoms, and may involve chronic pain (chronic pain refers to long-lasting pain, persisting for more than a few months, for example more than 3 months, or more than 6 months). For example, patients suffering from painful peripheral neuropathy may experience shooting and/or burning pain, and the like. Painful peripheral neuropathy can involve allodynia. Allodynia refers to pain elicited by a stimulus that, in the case of a healthy subject (as opposed to a subject experiencing allodynia, such as allodynia resulting from peripheral neuropathy) would not normally cause pain. Painful peripheral neuropathy can involve painful dysesthesia. Painful dysesthesia involves a painful, unpleasant and abnormal sensation of touch, for example sensations such as burning of the skin and/or sensations of electric shock. Painful peripheral neuropathy can involve episodic neuropathic pain or continuous neuropathic pain. Episodic neuropathic pain refers to recurring pain, typically involving periods of pain (for example, paroxysmal pain) interspersed with periods of relative comfort wherein the pain is alleviated. Continuous neuropathic pain refers to neuropathic pain that is substantially constant, and typically chronic.

Peripheral neuropathy affecting sensory nerves can be non-painful. Non-painful peripheral neuropathy refers to peripheral neuropathy resulting in non-painful symptoms, and may involve chronic non-painful symptoms (chronic non-painful symptoms refer to long-lasting non-painful symptoms, persisting for more than a few months, for example more than 3 months, or more than 6 months). For example, patients suffering from non-painful peripheral neuropathy may experience one or more of reduced sensitivity to temperature changes, numbness to touch and vibration, non-painful spontaneous tingling sensations, and the like; particularly reduced sensitivity to temperature changes. Non-painful peripheral neuropathy can involve episodic non-painful sensations or continuous non-painful sensations. Episodic non-painful sensations refer to (non-painful) sensations, typically involving periods of spontaneous sensations (for example, paroxysms of spontaneous sensations) interspersed with periods of relative comfort wherein the sensations are alleviated. Continuous non-painful sensations refer to non-painful sensations that are substantially constant, and typically chronic.

### Diabetic peripheral neuropathy (DPN)

Diabetic peripheral neuropathy (DPN) refers to peripheral neuropathy, whether painful or non-painful, diagnosed as induced by diabetes mellitus. For example, a patient suffering from diabetes mellitus may experience the signs and symptoms of diabetic peripheral neuropathy in the region of the foot.

Diabetic peripheral neuropathy can, for example, lead to symptoms such as pain, numbness, tingling and reduced sensitivity of the skin to temperature changes, for example the skin of the hands and/or feet, particularly the feet. Whether DPN develops, and its severity, may depend on the type of diabetes. For example, whether DPN develops may depend on whether the patient is suffering from type 1 diabetes, type 2 diabetes, or gestational diabetes. Whether DPN develops, and its severity, may additionally or alternatively depend on whether, and to what extent, the diabetes is controlled. For example, whether DPN develops, and its severity, may depend on whether the diabetes is poorly controlled or uncontrolled. Whether DPN develops, and its severity, may depend on how long a patient has been suffering from diabetes. For example, a patient who has been suffering from diabetes for more than 5, 10, 15 or 20 years may be more likely to develop DPN than a patient who has been suffering from diabetes for less than 5, 4, 3, or 2 years, or less than 1 year.

### Diabetic ulcers

Diabetic ulcers, for example diabetic foot ulcers, are a complication of diabetes. A diabetic ulcer, for example a diabetic foot ulcer, may be a surface ulcer, which is present through the thickness of the skin, but does not involve underlying tissues. A diabetic ulcer, for example a diabetic foot ulcer, may be a deep ulcer, which penetrates past the skin surface, to the ligaments and muscle, but does not involve abscess or bone. A diabetic ulcer, for example a diabetic foot ulcer, may be a deep ulcer involving inflammation of subcutaneous connective tissue, i.e. an abscess. Deep ulcers, for example deep diabetic foot ulcers, may include infections in the muscle, tendon, joint and/or bone. The tissue around the area of the ulcer may begin to decay, i.e. there may be necrotic tissue and/or gangrene. The gangrene may spread from the localised area of the ulcer.

One or more diabetic ulcers may occur on one or more limbs of a patient. For example, one or more diabetic ulcers may occur on the lower limb of a patient, such as on the foot of a patient. The size, shape and depth of a diabetic ulcer, for example a diabetic foot ulcer, may vary. A diabetic ulcer, for example a diabetic foot ulcer, may have a surface area of more than 1 cm², more than 2 cm², more than 3 cm², more than 4 cm², more than 5 cm², more than 6 cm², more than 7 cm², more than 8 cm², more than 9 cm², more than 10 cm², more than 11 cm², more than 12 cm², more than 13 cm², more than 14 cm², more than 15 cm², more than 16 cm², more than 17 cm², more than 18 cm², more than 19 cm², or more than 20 cm².

A diabetic ulcer, for example a diabetic foot ulcer, may develop and persist over a period of time. For example, a diabetic ulcer, such as a diabetic foot ulcer, may develop and persist over a period of more than 1 month, more than 2 months, more than 3 months, more than 4 months, more than 5 months, more than 6 months, more than 12 months, more than 18 months, or more than 24 months.

### Capsaicin

A molecule of capsaicin (8-methyl-N-vanillyl-6-nonenamide; IUPAC name *(6E)-N-[(4-Hydroxy-3-methoxyphenyl)methyl]-8-methylnon-6-enamide)* has the following structure:

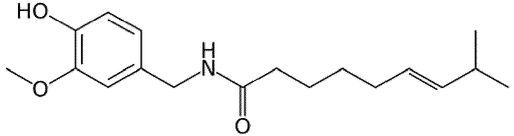

Capsaicin is a highly selective agonist for the transient receptor potential vanilloid 1 receptor (TRPV1). Capsaicin is found in some plants belonging to the genus *Capsicum,* which are members of the nightshade family, *Solanaceae.* Capsaicin is typically found in the fruit of some (but not all) of the plants belonging to the genus *Capsicum* that are commonly known as chili peppers. Capsaicin is typically present in these plants alongside other capsaicin derivatives known as capsaicinoids. The capsaicinoids in accordance with the claims herein are dihydrocapsaicin, nordihydrocapsaicin, homocapsaicin, capsiate, nonivamide, and homodihydrocapsaicin. For example, *Capsicum chinense* is a species of chili pepper that includes capsaicin-containing varieties, such as habanero, Datil and Scotch bonnet.

### Capsaicinoids

Capsaicinoids are a group of compounds with similar structure and function as capsaicin. As noted above, the capsaicinoids in accordance with the claims herein are dihydrocapsaicin, nordihydrocapsaicin, homocapsaicin, homodihydrocapsaicin, capsiate, and nonivamide. Certain preferred embodiments of the invention relate to capsaicin or dihydrocapsaicin (i.e., dihydrocapsaicin is the preferred capsaicinoid). In many embodiments of all aspects of the invention capsaicin is the preferred compound (i.e., capsaicin in preferred over an alternative capsaicinoid).

### Other TRPV1 agonists

These include Resiniferatoxin (RTX), a chemical in cactus-like plants from Africa (resin spurge, *Euphorbia resinifera, Euphorbia poissonii*) and derivatives thereof. According to certain embodiments of all aspects of the invention a compound is regarded as a TRPV1 agonist if it activates TRPV1. That activation may be measured using any suitable assay for example a patch-clamp assay. Suitable assays are routine as evidenced by the fact that they are available commercially on a contract basis for example from SB Drug Discovery Ltd, Glasgow, UK. According to certain embodiments, TRPV1 agonists show at least 20% or at least 50% or at least 100% or at least 200% of TRPV1 activation as does capsaicin in an identical assay. It is especially preferred if the TRPV1 agonist is fat soluble.

These include Resiniferatoxin (RTX), has the IUPAC name [(1*R*,6*R*,13*R*,15*R*,17*R*)-13-Benzyl-6-hydroxy-4,17-dimethyl-5-oxo-15-(prop-1-en-2-yl)-12,14,18-trioxapentacyclo[11.4.1.0^{1,10}.0^{2,6}.0^{11,15}]octadeca-3,8-dien-8-yl]methyl 2-(4-hydroxy-3-methoxyphenyl)acetate. A molecule of Resiniferatoxin has the following structure:

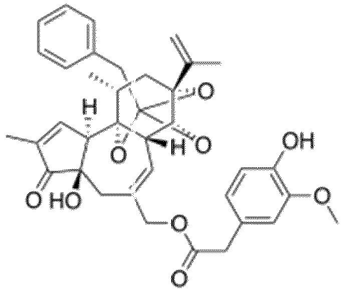

TRPV1 agonists also include phenylacetylrinvanil and capsazepine.

### Capsaicin 8% Patch

"Capsaicin 8% Patch" (Capsaicin 179 mg cutaneous patch) is the nonproprietary name for a cutaneous patch comprising capsaicin, which is also sold under the trade name Qutenza^{®}. Capsaicin 8% Patch is obtainable from Grünenthal Ltd, Units 1 and 2 Stokenchurch Business Park, Ibstone Road, Stokenchurch, Buckinghamshire, HP14 3FE, United Kingdom. Capsaicin 8% Patch consists of a 280 cm² cutaneous patch, which contains a total of 179 mg of capsaicin, that is, 640 µg of capsaicin per cm² patch. Capsaicin 8% Patch measures 14 cm x 20 cm (280 cm²) and consists of an adhesive side and an outer surface backing layer, the capsaicin-containing patch matrix being disposed on the adhesive side. Capsaicin 8% Patch comprises 8% capsaicin by weight, wherein the % by weight of capsaicin is measured relative to the total weight of all of the components in the patch matrix. The adhesive side is covered with a removable, clear, unprinted, diagonally cut, release liner. The outer surface of the backing layer is imprinted with "capsaicin 8%".

The patch matrix typically comprises silicone adhesives, diethylene glycol monoethyl ether, silicone oil, and ethylcellulose N50. The patch backing layer is typically formed from a polyethylene terephthalate (PET) film. The patch removable protective layer (release liner) is typically formed from a fluoropolymer-coated polyester film.

### Nerve fibre regeneration and/or restoration

Nerve fibre regeneration refers to the sprouting and elongation of nerve fibres which have been previously damaged or destroyed. Nerve fibre regeneration can lead to the full or partial restoration of the density and phenotype of one or more nerve cells (for example, nerve cells of the leg/foot) to a normalised, healthy condition, compared to the abnormal, damaged condition of nerve cells observed in patients suffering from diabetic peripheral neuropathy. Nerve fibre regeneration and restoration can potentially lead to the full or partial restoration of the function of one or more nerve cells (for example, nerve cells of the leg/foot) to a normalised, healthy condition, compared to the abnormal, damaged condition of nerve cells observed in patients suffering from diabetic peripheral neuropathy. Nerve fibre regeneration and restoration can lead to the full or partial restoration of at least one or more of the following functions of one or more nerve cells (for example, nerve cells of the leg/foot) to a normalised, healthy condition:
protective sensation (the full or partial restoration of which is thought to alert the patient to skin tissue damage at the earliest possible stage, thus helping to prevent ulcer formation, such as foot ulcer formation);
supporting the dilation of the vasculature of the skin in response to the application of skin surface pressure (as well as in response, for example, to heat and/or chemical stimulants, again helping to prevent ulcer formation, such as foot ulcer formation); and
regulation of the trophic response required for the maintenance of healthy skin tissue (to help forestall tissue damage, which otherwise can lead to a risk of ulcer formation) and/or of the trophic response required for the healing of damaged skin tissue (it is thought that the full or partial restoration of an appropriate trophic response following damage to skin tissue can help prevent ulcer formation, for example by ensuring rapid wound healing at an early stage of injury, so that initial, minor damage to skin tissue does not progress to a more serious injury, such as an ulcer, for example a foot ulcer).

According to preferred embodiments of all aspects of the invention, nerve fibre regeneration is understood to be the regeneration of a nerve fibre belonging to an existing nerve cell, for example an existing nerve cell of the leg/foot. That is to say, nerve fibre regeneration does not necessarily require an increase in the number of nerve cells. Rather, existing nerve cells, for example, nerve cells of the leg/foot, which may have previously lost a region of nerve fibre, may see that region of nerve fibre regrow. Nerve fibre regeneration also includes the restoration of density, phenotype and potentially function (such as protective sensation, and particularly supporting an appropriate vascular response to skin surface pressure or heat, in addition to regulation of the trophic response required for the maintenance of healthy skin tissue, and/or of the trophic response required for the healing of damaged skin tissue) of a previously dysfunctional nerve fibre (for example, a previously dysfunctional nerve fibre of the leg/foot) i.e. which generated pain sensations. In certain embodiments, nerve fibre regeneration may especially include the restoration of functional nerve fibre terminals, for example nerve fibre terminals of the leg/foot.

Nerve fibre regeneration, for example the regeneration of nerve fibres of the leg/foot, can be measured by immunohistochemical analysis of skin biopsies. A statistically significant increase in the density of intra-epidermal and sub-epidermal nerve fibres, observed in immunohistochemical analysis of skin biopsies, can indicate nerve fibre regeneration, for example the regeneration of nerve fibres of the leg/foot. A statistically significant increase (for example an increase of at least 10, 20, 30, or 50%) in the count of sub-epidermal nerve fibres, observed in immunohistochemical analysis of skin biopsies, can indicate nerve fibre regeneration in accordance with the invention, for example the regeneration of nerve fibres of the leg/foot. A statistically significant increase in the count of both intra-epidermal nerve fibres and sub-epidermal nerve fibres, observed in immunohistochemical analysis of skin biopsies, can indicate nerve fibre regeneration, for example the regeneration of nerve fibres of the leg/foot.

For example, a statistically significant increase in the density of PGP9.5-immunostained intra-epidermal nerve fibres, observed in immunohistochemical analysis of skin biopsies, can indicate nerve fibre regeneration in accordance with the invention, such as the regeneration of nerve fibres of the foot. Similarly, a statistically significant increase in the count of PGP9.5-immunostained sub-epidermal nerve fibres, observed in immunohistochemical analysis of skin biopsies, can indicate nerve fibre regeneration, such as the regeneration of nerve fibres of the leg/foot. A statistically significant increase in the count of both PGP9.5-immunostained intra-epidermal nerve fibres and PGP9.5-immunostained sub-epidermal nerve fibres, observed in immunohistochemical analysis of skin biopsies, can indicate nerve fibre regeneration in accordance with the invention, such as the regeneration of nerve fibres of the leg/foot.

A statistically significant increase in the count of GAP43-immunostained sub-epidermal nerve fibres, observed in immunohistochemical analysis of skin biopsies, can indicate nerve fibre regeneration in accordance with the invention, such as the regeneration of nerve fibres of the leg/foot.

It will be appreciated that an increase or decrease in density described herein may be measured relative to the density observed in immunohistochemical analysis of skin biopsies in a subject having nerve fibres (for example, nerve fibres of the foot) which are damaged or destroyed, such as a subject who is suffering from, and has not received treatment for, diabetic peripheral neuropathy.

It will be appreciated that a statistically significant change in density or level described herein can refer to the density or level approaching or reaching the count or level observed in a control subject not suffering from diabetic peripheral neuropathy.

### Detailed Description

As noted above and as defined in claim 1, the present invention provides capsaicin, dihydrocapsaicin, nordihydrocapsaicin, homocapsaicin, homodihydrocapsaicin, capsiate, nonivamide or a TRPV1 agonist for use in the prevention of diabetic ulcers, in a patient suffering from diabetic peripheral neuropathy, wherein the capsaicin dihydrocapsaicin, nordihydrocapsaicin, homocapsaicin, homodihydrocapsaicin, capsiate, nonivamide or TRPV1 agonist is administered topically to one or more areas of the skin. Further optional features are defined in the dependent claims.

It will be understood that any reference to methods of treatment of the human (or animal) body by therapy in this description is to be construed as reference to the compounds, pharmaceutical compositions and medicaments of the present invention for use in such a method of treatment.

### Peripheral neuropathy, diabetic peripheral neuropathy

Peripheral neuropathy refers to a condition wherein the peripheral nerves (nerves that lie beyond the brain and spinal cord) are damaged. In general, peripheral neuropathy can involve damage to one or more sensory, autonomic, and motor nerves. Certain preferred embodiments of all aspects of the present invention concern peripheral neuropathy of sensory nerves, particularly sensory nerves of the skin (i.e. sensory neurons which terminate between or adjacent to skin cells, that is to say, nerve cells having cutaneous sensory nerve terminals) for example sensory nerves of the skin of the leg/foot. Diabetic peripheral neuropathy can comprise painful and/or non-painful diabetic peripheral neuropathy, and typically involves damage to the sensory nerves, for example the sensory nerves of the leg/foot.

In some embodiments of the uses of the present invention, diabetic peripheral neuropathy includes painful peripheral neuropathy. In some embodiments, diabetic peripheral neuropathy includes non-painful peripheral neuropathy. In some embodiments, diabetic peripheral includes both painful peripheral neuropathy and non-painful peripheral neuropathy.

Examples of symptoms and signs of diabetic peripheral neuropathy according to the invention include, but are not limited to: reduced sensitivity of the skin to temperature changes; sensations of numbness to touch and vibration; sensations of non-painful spontaneous tingling; and sensations of pain, for example burning pain, sharp, stabbing pain and sensations of receiving an electric shock. For example, such symptoms and signs of diabetic peripheral neuropathy, such as reduced sensitivity of the skin to temperature changes, may be observed in the region of the leg/foot. According to certain embodiments, the diabetic peripheral neuropathy in relation to any aspect of the present invention is chronic diabetic peripheral neuropathy (i.e., diabetic peripheral neuropathy wherein clinical symptoms are exhibited for at least 1, 3 or 6 months) for example chronic painful or non-painful diabetic peripheral neuropathy wherein symptoms and signs of diabetic peripheral neuropathy are observed in the leg/foot.

### Capsaicin or capsaicinoid or TRPV1 agonist effective amount

According to all aspects of the invention, the capsaicin or capsaicinoid (which, it will be understood, is in accordance with the claims: dihydrocapsaicin, nordihydrocapsaicin, homocapsaicin, homodihydrocapsaicin, capsiate or nonivamide) or TRPV1 agonist is provided in a therapeutically effective amount (for example, about 350 to about 1000 µg of capsaicin per cm² of skin, such as about 500 to about 700 µg of capsaicin per cm² of skin). The amount of capsaicin or capsaicinoid or TRPV1 agonist which is required to achieve a therapeutic effect will vary with the subject under treatment, including the age, weight, sex, and medical condition of the subject. The amount of capsaicin or capsaicinoid or TRPV1 agonist which is required to achieve a therapeutic effect can vary with the type of diabetes, for example whether it is type 1 diabetes, type 2 diabetes, or gestational diabetes. The amount of capsaicin or capsaicinoid or TRPV1 agonist which is required to achieve a therapeutic effect may vary depending on whether, and to what extent, the diabetes is controlled, for example whether it is poorly controlled or uncontrolled. The amount of capsaicin or capsaicinoid or TRPV1 agonist which is required to achieve a therapeutic effect may vary depending on the type (painful or non-painful) and severity of the diabetic peripheral neuropathy.

It will be appreciated that capsaicin or a capsaicinoid or a TRPV1 agonist may achieve a therapeutic effect in the uses of the present invention.

### Administering capsaicin or a capsaicinoid

According to all aspects of the present invention, administering capsaicin or a capsaicinoid (which, it will be understood, is in accordance with the claims: dihydrocapsaicin, nordihydrocapsaicin, homocapsaicin, homodihydrocapsaicin, capsiate or nonivamide) or a TRPV1 agonist involves administering capsaicin or a capsaicinoid or a TRPV1 agonist topically to one or more areas of the skin of the patient, for example to one or more areas of the skin of the foot.

Examples of topical formulations that can be used for the administration of capsaicin or a capsaicinoid or a TRPV1 agonist include, but are not limited to: creams, foams, gels, lotions, ointments, pastes, powders, tinctures, and cutaneous patches. It should be understood that the topical formulations for use in the administration of capsaicin or a capsaicinoid or a TRPV1 agonist may include excipients conventional in the art.

Administering capsaicin topically to one or more areas of skin, for example one or more areas of the skin of the foot, can comprise administering from about 200 to about 1000 µg of capsaicin per cm² of skin; from about 250 to about 1000 µg of capsaicin per cm² of skin; from about 300 to about 1000 µg of capsaicin per cm² of skin; from about 350 to about 1000 µg of capsaicin per cm² of skin; from about 400 to about 1000 µg of capsaicin per cm² of skin; from about 500 to about 1000 µg of capsaicin per cm² of skin; from about 500 to about 700 µg of capsaicin per cm² of skin; from about 550 to about 700 µg of capsaicin per cm² of skin; from about 600 to about 700 µg of capsaicin per cm² of skin; or from about 620 to about 660 µg of capsaicin per cm² of skin. For example, administering capsaicin topically to one or more areas of skin, such as one or more areas of the skin of the foot, can comprise administering from about 350 to about 1000 µg of capsaicin per cm² of skin, such as about 500 to about 700 µg of capsaicin per cm² of skin, for example about 640 µg of capsaicin per cm² of skin. Similar dosages of another capsaicinoid or another TRPV1 agonist can be administered topically to one or more areas of skin, for example one or more areas of the skin of the foot. Alternatively, the dosage of other capsaicinoids or TRPV1 agonists can be adjusted to take into account differential therapeutic activity levels per molecule relative to capsaicin. For dihydrocapsaicin, the dosage may not need to be adjusted, and the dosages stated above in relation to capsaicin may apply. For the other capsaicinoids the dosage may optionally be increased by about 80% relative to the dosages stated above for capsaicin.

The one or more areas of skin, for example one or more areas of the skin of the foot, to which capsaicin or a capsaicinoid or TRPV1 agonist is administered can have a total surface area of from about 5 to about 1120 cm²; from about 10 to about 1120 cm²; from about 20 to about 1120 cm²; from about 50 to about 1120 cm²; from about 75 to about 1120 cm²; from about 100 to about 1120 cm²; from about 100 to about 800 cm²; from about 100 to about 600 cm²; from about 100 to about 500 cm²; or from about 100 to about 400 cm². It will be appreciated that the one or more areas of skin, for example one or more areas of the skin of the foot, to which capsaicin or a capsaicinoid or TRPV1 agonist is administered can differ from patient to patient, depending on their symptoms and signs of diabetic peripheral neuropathy and the extent of the diabetic peripheral neuropathy across the body surface. The one or more areas of skin, for example one or more areas of the skin of the foot, to which capsaicin or a capsaicinoid or TRPV1 agonist is administered can be one or more areas of skin, for example one or more areas of the skin of the foot, that exhibit symptoms and/or signs of diabetic peripheral neuropathy. An ordinarily skilled physician can readily determine the one or more areas of skin, for example one or more areas of the skin of the foot, for exposure to capsaicin or a capsaicinoid or TRPV1 agonist, for example by touching different areas of skin (or instructing the patient to do that to themselves) and asking the patient whether each area of the skin has symptoms. It may be a useful tool for the planning of the treatment, for record keeping, and for monitoring the treatment for this information to be illustrated on a diagram of the body. Additionally or alternatively, this information may be traced directly onto skin of the patient, using for example an ink pen to mark the skin. Alternatively, or additionally, more sensitive or more accurate or more objective information as to the extent of the diabetic peripheral neuropathy may be obtained by using neurophysiological testing, whereby a sensory perception threshold or nerve conduction device is used to assess nerve fibre function.

In some embodiments, administering capsaicin or a capsaicinoid or TRPV1 agonist topically comprises administering capsaicin or a capsaicinoid formulated in a cutaneous patch to one or more areas of the skin of the patient, for example one or more areas of the skin of the foot. A cutaneous patch can comprise an adhesive side and an outer surface backing layer, and can have a capsaicin-containing or a capsaicinoid-containing or a TRPV1 agonist-containing patch matrix disposed on the adhesive side. The adhesive side can be covered with a removable release liner.

A cutaneous patch for administering capsaicin or a capsaicinoid or a TRPV1 agonist can have a surface area of from about 5 to about 1120 cm²; from about 10 to about 1120 cm²; from about 20 to about 1120 cm²; from about 50 to about 1120 cm²; from about 75 to about 1120 cm²; from about 100 to about 500 cm²; from about 200 to about 400 cm²; from about 250 to about 350 cm²; from about 250 to about 300 cm²; or from about 270 to about 290 cm². For example, a cutaneous patch for administering capsaicin or a capsaicinoid or TRPV1 agonist can have a surface area of about 280 cm². It will be appreciated that a patch may be cut to size, to suit the requirements of an individual patient. It should also be understood that two, three, four, five, six or more patches can be applied simultaneously to the skin of the patient, for example to one or more areas of the skin of the foot, to suit the requirements of an individual patient. An ordinarily skilled physician can readily determine and prescribe an effective patch size required for exposure of one or more areas of the skin, for example one or more areas of the skin of the foot, to capsaicin or a capsaicinoid or a TRPV1 agonist. The physician may also find it useful to trim a relatively large patch in order to apply it to a relatively small area of skin, for example a relatively small area of the skin of the foot. As such, patches of the invention are preferably arranged to be easily cut with scissors.

Capsaicin and capsaicinoids, and also certain preferred TRPV1 agonists, are highly lipid soluble and minimally water soluble. In consequence, when applied to unbroken skin, very little of the compound enters the circulation. Thus, therapeutic effects are largely localised. It is likely that a certain amount of the compound will dissolve in skin fat, for example fat of the foot skin (when the patch is applied to one or more areas of the skin of the foot) and that this will provide something of a depository effect, such that the target nerve fibres (for example, target nerve fibres of the skin of the foot) will continue to be exposed to the compound even after the patch has been removed.

The matrix of a cutaneous patch for administering capsaicin can comprise from about 1 to about 20 % by weight capsaicin; from about 3 to about 20 % by weight capsaicin; more preferably, from about 5 to about 20 % by weight capsaicin; from about 5 to about 15 % by weight capsaicin; from about 5 to about 10% by weight capsaicin; or from about 6 to about 10 % by weight capsaicin. For example, the matrix of a cutaneous patch for administering capsaicin can comprise about 8 % by weight capsaicin. It will be appreciated that the % by weight of capsaicin is measured relative to the total weight of the patch matrix, and that capsaicin is typically evenly distributed throughout the patch matrix. The matrix of a cutaneous patch for administering capsaicin can comprise from about 200 to about 1000 µg of capsaicin per cm² of patch; from about 250 to about 1000 µg of capsaicin per cm² of patch; from about 300 to about 1000 µg of capsaicin per cm² of patch; from about 350 to about 1000 µg of capsaicin per cm² of patch; from about 400 to about 1000 µg of capsaicin per cm² of patch; from about 500 to about 1000 µg of capsaicin per cm² of patch; from about 500 to about 700 µg of capsaicin per cm² of patch; from about 550 to about 700 µg of capsaicin per cm² of patch; from about 600 to about 700 µg of capsaicin per cm² of patch; or from about 620 to about 660 µg of capsaicin per cm² of patch. For example, the matrix of a cutaneous patch for administering capsaicin can comprise from about 350 to about 1000 µg of capsaicin per cm² of patch, such as about 500 to about 700 µg of capsaicin per cm² of patch, for example about 640 µg of capsaicin per cm² of patch.

Similar dosages of another capsaicinoid or another TRPV1 agonist can be administered topically to one or more areas of skin, for example one or more areas of the skin of the foot. Alternatively, the dosage of other capsaicinoids or TRPV1 agonists can be adjusted to take into account differential therapeutic activity levels per molecule relative to capsaicin. For dihydrocapsaicin, the dosage may not need to be adjusted and the dosages stated above in relation to capsaicin may apply. For the other capsaicinoids, the dosage may optionally be increased by about 80% relative to the dosages stated above for capsaicin.

The patch matrix can comprise excipients. Examples of suitable excipients for use in a patch matrix include, but are not limited to: silicone adhesives, diethylene glycol monoethyl ether, silicone oil, and ethylcellulose N50. It should be understood that the patch matrix may include further excipients conventional in the art. The patch backing layer can be formed from a polyethylene terephthalate (PET) film. The patch removable release liner can be formed from a fluoropolymer-coated polyester film.

For reasons of safety, capsaicin and capsaicinoids and TRPV1 agonists should not normally be administered to the skin of, or near, the eyes. Likewise, capsaicin and capsaicinoids and TRPV1 agonists should not normally be administered to mucous membranes. Capsaicin and capsaicinoids and TRPV1 agonists should not normally be administered to the skin of the head, and in particular capsaicin and capsaicinoids and TRPV1 agonists should not normally be administered to the skin of the face. Capsaicin and capsaicinoids and TRPV1 agonists should not normally be administered to broken, irritated or otherwise sensitive areas of skin, for example the skin of the anogenital regions of the body.

The one or more areas of skin, for example one or more areas of the skin of the foot, to which capsaicin or a capsaicinoid or TRPV1 agonist is administered can be pre-treated with a topical anaesthetic. Alternatively, or additionally, the one or more areas of skin, for example one or more areas of the skin of the foot, to which capsaicin or a capsaicinoid or TRPV1 agonist is administered, may be simultaneously treated with a topical anaesthetic. Accordingly, the invention in all its aspects relates to compositions containing as their active ingredient a topical anaesthetic and capsaicin or a capsaicinoid or TRPV1 agonist. Additionally, or alternatively, the patient may be administered an oral analgesic immediately prior to topical administration of capsaicin or a capsaicinoid or TRPV1 agonist. For example, the patient may receive topical pre-treatment with lidocaine, prilocaine or both lidocaine and prilocaine. Normally, any topical anaesthetics can then be removed prior to topical administration of capsaicin or a capsaicinoid or TRPV1 agonist, and the one or more areas of skin, for example one or more areas of the skin of the foot, can be washed and dried thoroughly, although it may in certain embodiments be desirable to administer the capsaicin or capsaicinoid or TRPV1 agonist simultaneously (in such cases, suitable compositions may be formulated and provided). Additionally, or alternatively, a cold pack can be administered before, during or after topical administration of capsaicin or capsaicinoid or TRPV1 agonist. Hairs in the one or more areas of skin, for example one or more areas of the skin of the foot, can be clipped or otherwise removed before administering capsaicin or a capsaicinoid or TRPV1 agonist topically, but should normally not be shaved (in order to avoid breaking the skin). The treatment area(s) can then be gently washed with soap and water, then dried, before administering capsaicin or a capsaicinoid or a TRPV1 agonist topically.

Capsaicin or a capsaicinoid or a TRPV1 agonist can be administered topically to one or more areas of skin, for example one or more areas of the skin of the foot, then allowed to remain in place for a period of time. In some embodiments, administering capsaicin or a capsaicinoid or a TRPV1 agonist topically comprises administering capsaicin or a capsaicinoid or a TRPV1 agonist to one or more areas of skin, for example one or more areas of the skin of the foot, for a period of about 1 to about 90 minutes; about 10 to about 90 minutes; about 20 to about 90 minutes; about 30 to about 90 minutes; about 10 to about 80 minutes; about 20 to about 80 minutes; about 30 to about 80 minutes; about 10 to about 70 minutes; about 20 to about 70 minutes; about 30 to about 70 minutes; about 10 to about 60 minutes; about 20 to about 60 minutes; about 30 to about 60 minutes; about 10 to about 50 minutes; about 20 to about 50 minutes; or about 30 to about 50 minutes. For example, administering capsaicin or a capsaicinoid or a TRPV1 agonist topically can comprise administering capsaicin or a capsaicinoid or a TRPV1 agonist to one or more areas of skin, for example one or more areas of the skin of the foot, for a period of about 30 to about 60 minutes. An ordinarily skilled physician can readily determine and prescribe an effective length of time required for exposure of one or more areas of the skin, for example one or more areas of the skin of the foot, to capsaicin or a capsaicinoid or TRPV1 agonist. As a point of general guidance, skin with a higher level of fat, in particular skin of the plantar surface of the foot, may need a shorter application of capsaicin or a capsaicinoid due to the fat soluble nature of capsaicin and capsaicinoids and of many TRPV1 agonists. As a starting point, approximately 60 minutes may be a suitable period of time for most areas of the skin, and approximately 30 minutes may be a suitable period of time for the plantar surface of the feet.

In some embodiments, capsaicin or a capsaicinoid or a TRPV1 agonist can be administered topically to more than one area of the skin, for example more than one area of the skin of the foot, simultaneously. In some embodiments, capsaicin or a capsaicinoid or a TRPV1 agonist can be administered topically to different areas of the skin, for example different areas of the skin of the foot, sequentially. An appropriate time interval between sequential topical administrations of capsaicin or a capsaicinoid or a TRPV1 agonist to different areas of the skin, for example different areas of the skin of the foot, can be about 4 weeks, about 5 weeks, about 6 weeks, about 7 weeks, about 8 weeks, about 9 weeks, about 10 weeks, about 11 weeks, about 12 weeks, or more. In some embodiments, capsaicin or a capsaicinoid or a TRPV1 agonist can be administered topically to the same area of the skin, for example the same area of the skin of the foot, sequentially. An appropriate time interval between sequential topical administrations of capsaicin or a capsaicinoid or a TRPV1 agonist to the same area of the skin, for example the same area of the skin of the foot, can be about 4 weeks, about 5 weeks, about 6 weeks, about 7 weeks, about 8 weeks, about 9 weeks, about 10 weeks, about 11 weeks, about 12 weeks, or more. An ordinarily skilled physician can readily determine and prescribe an effective time interval between sequential topical applications of capsaicin or a capsaicinoid or a TRPV1 agonist to the same, or different, areas of the skin (for example of the skin of the foot).

After removal of topically administered capsaicin or a capsaicinoid or a TRPV1 agonist, cleansing gel can be applied to the one or more areas of the skin, for example one or more areas of the skin of the foot. The cleansing gel can be left in place for about 30 to about 120 seconds; for about 60 to about 120 seconds; or for about 60 to about 90 seconds. The cleansing gel can then be removed. Removal of the cleansing gel can comprise wiping the cleansing gel from the skin surface, using dry gauze, in order to remove any remaining capsaicin or capsaicinoid from the skin. After the cleansing gel has been wiped off, the one or more areas of skin, for example one or more areas of the skin of the foot, can be gently washed with soap and water. Optionally, the kit may additionally comprise one or more items selected from cleaning gel, gauze, soap and personal protective equipment such as gloves.

An appropriate cleansing gel, for use in cleansing one or more areas of skin, for example one or more areas of the skin of the foot, to which capsaicin or a capsaicinoid or TRPV1 agonist has been applied, is a cleansing gel comprising about 0.05 to about 0.5 mg/g butylhydroxyanisole; about 0.05 to about 0.45 mg/g butylhydroxyanisole; about 0.05 to about 0.4 mg/g butylhydroxyanisole; or about 0.1 to about 0.3 mg/g butylhydroxyanisole. For example, an appropriate cleansing gel can comprise about 0.2 mg/g butylhydroxyanisole. Optionally, the cleansing gel can also contain excipients, including but not limited to: macrogol 300, carbomer, purified water, sodium hydroxide and disodium edetate.

In some embodiments, administering capsaicin or a capsaicinoid or a TRPV1 agonist topically comprises administering Capsaicin 8% Patch (ie, Qutenza^{®} or a generic equivalent thereof) to one or more areas of the skin of the patient, for example to one or more areas of the skin of the foot. Administering Capsaicin 8% Patch can comprise applying Capsaicin 8% Patch to an area of skin, for example an area of the skin of the foot, and allowing it to remain in place for a period of time. Where the area of skin is an area of the skin of the feet, Capsaicin 8% Patch can be allowed to remain in place for a period of time of about 20 to about 40 minutes, such as about 25 to about 35 minutes (e.g., about 30 minutes). Where the area of the skin is not an area of skin of the feet, it is typically allowed to remain in place for about 50 to about 70 minutes, such as about 55 to about 65 minutes (e.g. about 60 minutes). In some embodiments wherein Capsaicin 8% Patch is administered to the skin of the patient, Capsaicin 8% Patch can be administered to one or more areas of the skin of the patient, for example one or more areas of the skin of the foot, for a period of about 30 minutes to about 60 minutes.

The one or more areas of skin, for example one or more areas of the skin of the foot, to which Capsaicin 8% Patch is administered can be pre-treated with a topical anaesthetic. Additionally, or alternatively, the patient may be administered an oral analgesic immediately prior to topical administration of Capsaicin 8% Patch. For example, the patient may receive pre-treatment with lidocaine, prilocaine or both lidocaine and prilocaine. Any topical anaesthetics can then be removed prior to administration of Capsaicin 8% Patch, and the area of the skin, for example an area of the skin of the foot, can be washed and dried thoroughly. Additionally, or alternatively, a cold pack can be administered before, during or after topical administration of Capsaicin 8% Patch. Hairs in the area of the skin can be clipped or otherwise removed before administering Capsaicin 8% Patch. The treatment area(s) can then be gently washed with soap and water, then dried, before administering Capsaicin 8% Patch. Additionally, or alternatively, a cold pack can be administered before, during or after patch application, to reduce any pain induced by Capsaicin 8% Patch.

Capsaicin 8% Patch should not be used on or near the eyes or mucous membranes. Capsaicin 8% Patch should not be applied to the skin of the head. In particular, Capsaicin 8% Patch should not be applied to the skin of the face. Capsaicin 8% Patch should not be applied to broken, irritated or otherwise sensitive areas of skin such as the anogenital area of the body.

Hairs in the area of the skin, for example an area of the skin of the foot, can be clipped, or otherwise removed, before applying Capsaicin 8% Patch, in order to promote patch adherence (although the skin should not be shaved, in order to avoid breaking the skin). If hairs are removed in this way, the treatment area(s) can then be gently washed with soap and water, then thoroughly dried.

It will be appreciated that Capsaicin 8% Patch is a single use patch. Capsaicin 8% Patch can be cut to match the size and shape of the area of skin, for example an area of skin of the foot. If the patch is to be cut in this manner, it should be cut prior to removal of the release liner. The release liner should not be removed until just prior to application. To apply the patch, a section of the release liner can be peeled away and folded, exposing the adhesive side of the patch. The adhesive side of the patch can then be placed on the area of skin, for example an area of the skin of the foot. The patch can then be held in place. The release liner can slowly and carefully be peeled from underneath, while the patch is simultaneously smoothed onto the skin, to ensure that there is contact between the patch and the skin. To ensure the patch remains in contact with the area of the skin during use, stretchable socks or rolled gauze can be applied over the patch to hold it in place.

Typically, Capsaicin 8% Patch is removed gently and slowly by rolling it inward, without the patch being allowed to come into contact with any other area of the skin.

After removal of Capsaicin 8% Patch, cleansing gel is typically applied to the area of the skin, for example the area of the skin of the foot, to which Capsaicin 8% Patch has been applied. The cleansing gel can be left in place for about 30 to about 120 seconds, such as for about 60 to about 120 seconds (e.g. for about 60 to about 90 seconds). The cleansing gel can then be removed. Removal of the cleansing gel typically comprises wiping the cleansing gel off the skin surface, using dry gauze, in order to remove any remaining capsaicin from the skin. After the cleansing gel has been wiped off, the area can be gently washed with soap and water.

An appropriate cleansing gel, for use in cleansing an area of the skin, for example an area of the skin of the foot, to which capsaicin has been applied, is a cleansing gel comprising about 0.05 to about 0.5 mg/g butylhydroxyanisole; about 0.05 to about 0.45 mg/g butylhydroxyanisole; about 0.05 to about 0.4 mg/g butylhydroxyanisole; or about 0.1 to about 0.3 mg/g butylhydroxyanisole. For example an appropriate cleansing gel can comprise about 0.2 mg/g butylhydroxyanisole. Optionally, the cleansing gel can also contain excipients, including but not limited to: macrogol 300, carbomer, purified water, sodium hydroxide and disodium edetate. It should be understood that the cleansing gel may include further excipients conventional in the art.

Optionally, a kit comprising Capsaicin 8% Patch may additionally comprise one or more items selected from cleaning gel, gauze, soap and personal protective equipment such as gloves.

### Clinical benefits

Without wishing to be bound by theory, it is thought that capsaicin, a capsaicinoid (which, it will be understood, is in accordance with the claims: dihydrocapsaicin, nordihydrocapsaicin, homocapsaicin, homodihydrocapsaicin, capsiate or nonivamide) or a TRPV1 agonist (particularly in high doses, for example about 350 to about 1000 µg per cm² of skin, such as about 500 to about 700 µg per cm² of skin) when administered to a patient suffering from diabetic peripheral neuropathy (whether painful or non-painful diabetic peripheral neuropathy; for example, when administered topically to one or more areas of the skin of the foot) induces degeneration of cutaneous sensory nerve terminals (for example, cutaneous sensory nerve terminals of the foot) followed by their regeneration. Regeneration of cutaneous sensory nerve terminals (for example, cutaneous sensory nerve terminals of the foot) can lead to the full or partial restoration of the density and phenotype of one or more nerve cells to a normalised, healthy condition, compared to the abnormal, damaged condition of nerve cells observed in patients suffering from diabetic peripheral neuropathy (whether painful or non-painful).

It is thought that improving sensory function, including sensitivity to temperature changes and other external stimuli, in patients with (painful and/or non-painful) diabetic peripheral neuropathy in this way can prevent the formation of diabetic ulcers, for example diabetic foot ulcers. The full or partial restoration of (normal) protective sensation through the regeneration and restoration of sensory nerve fibres is thought to alert the patient to skin tissue damage at the earliest possible stage, thus helping to prevent ulcer (e.g. foot ulcer) formation.

It is thought that regenerated peripheral nerves can help to regulate blood flow to areas of tissue subjected to pressure, so that adequate tissue perfusion is maintained and ulcers, such as foot ulcers, do not form. It is thought that the full or partial restoration of an appropriate vascular response to skin surface pressure may comprise the full or partial restoration of the ability of the vasculature of the skin to dilate in response to the application of skin surface pressure (as well as in response, for example, to heat and/or chemical stimulants). Thus, the re-establishment of a more normal circulatory response helps prevent ulcer (e.g. foot ulcer) formation.

It is also thought that regenerated peripheral nerves can help to regulate the trophic response required for the maintenance of healthy skin tissue, and/or the trophic response required for the healing of damaged skin tissue. In some embodiments, regenerated peripheral nerves may release growth factors responsible for an appropriate trophic response of skin cells. The full or partial restoration of the trophic response required to maintain healthy skin tissue, and/or of an appropriate trophic response following damage to skin tissue, can help prevent ulcer (e.g. foot ulcer) formation, as described herein. The full or partial restoration of the trophic response required to maintain healthy skin tissue, and/or of an appropriate trophic response following damage to skin tissue, may involve the full or partial restoration of an appropriate level of one or more growth factors in the tissue of the skin, for example one or more of nerve growth factor (NGF); glial cell line-derived neurotrophic factor (GDNF); epidermal growth factor (EGF); fibroblast growth factor (FGF); brain-derived neurotrophic factor (BDNF); and neurotrophin-3 (NT-3).

When one or more diabetic ulcers, particularly deep ulcers, develop on the limb of a patient (for example on the foot of the patient) this can necessitate amputation of the ulcer-bearing limb. Therefore, by preventing the formation of ulcers in the first place, the uses of the present invention can also help prevent any associated need for amputation.

Patients suffering from (painful and/or non-painful) diabetic peripheral neuropathy, when treated with capsaicin or a capsaicinoid or a TRPV1 agonist administered topically to one or more areas of the skin (for example, about 350 to about 1000 µg of capsaicin per cm² of skin, such as about 500 to about 700 µg of capsaicin per cm² of skin, optionally administered to one or more areas of the skin of the foot) can report significant changes in sensory perception, for example sensitivity to temperature changes, as measured by quantitative sensory testing (QST). For example, patients treated in accordance with the present invention can report a decrease in the threshold for warm sensory perception (i.e. improved sensation) over the course of about three months following treatment, as measured by QST, of from about 1.5 to about 3.5 °C, or from about 1.5 to about 2.5 °C.

Patients suffering from (painful and/or non-painful) diabetic peripheral neuropathy, when treated with capsaicin or a capsaicinoid or a TRPV1 agonist administered topically to one or more areas of the skin (for example, about 350 to about 1000 µg of capsaicin per cm² of skin, such as about 500 to about 700 µg of capsaicin per cm² of skin, optionally administered to one or more areas of the skin of the foot) can report significant spontaneous pain reduction, as measured by the Numerical Pain Rating Scale (NPRS). For example, patients treated in accordance with the present invention can report a reduction in spontaneous pain over the course of about three months following treatment, as measured by the NPRS, of from about 0.5 to about 2.5 points, or from about 1.0 to about 2.0 points.

Patients suffering from (painful and/or non-painful) diabetic peripheral neuropathy, when treated with capsaicin or a capsaicinoid or a TRPV1 agonist administered topically to one or more areas of the skin (for example, about 350 to about 1000 µg of capsaicin per cm² of skin, such as about 500 to about 700 µg of capsaicin per cm² of skin, optionally administered to one or more areas of the skin of the foot) can report significant pain reduction, as measured by the Short Form McGill Pain Questionnaire (SFMPQ). For example, patients treated in accordance with the present invention can report a reduction in intermittent pain over the course of about three months following treatment, as measured by the SFMPQ, of from about 3 to about 16 points, from about 6 to about 12 points, or from about 8 to about 10 points. Patients treated in accordance with the present invention can report a reduction in affective pain over the course of about three months following treatment, as measured by the SFMPQ, of from about 4 to about 14 points, from about 6 to about 12 points, or from about 8 to about 10 points. Patients treated in accordance with the present invention can report a reduction in neuropathic pain over the course of about three months following treatment, as measured by the SFMPQ, of from about 4 to about 12 points, from about 4 to about 10 points, or from about 4 to about 6 points. Patients treated in accordance with the present invention can report a reduction in overall pain over the course of about three months following treatment, as measured by the SFMPQ, of from about 1 to about 35 points, from about 1 to about 20 points, from about 1 to about 10 points, or from about 1 to about 5 points.

Patients suffering from (painful and/or non-painful) diabetic peripheral neuropathy, when treated with capsaicin or a capsaicinoid or a TRPV1 agonist administered topically to one or more areas of the skin (for example, about 350 to about 1000 µg of capsaicin per cm² of skin, such as about 500 to about 700 µg of capsaicin per cm² of skin, optionally administered to one or more areas of the skin of the foot) can report a significant general change in their condition following treatment with capsaicin or a capsaicinoid or a TRPV1 agonist, as measured by the Patient Global Impression of Change (PGIC). For example, patients treated in accordance with the present invention can report a change in PGIC score, over the course of about three months following treatment, of from about 0.3 to about 1.5, about 0.3 to about 0.8, or about 0.4 to about 0.7.

Patients suffering from (painful and/or non-painful) diabetic peripheral neuropathy, when treated with capsaicin or a capsaicinoid or a TRPV1 agonist administered topically to one or more areas of the skin (for example, about 350 to about 1000 µg of capsaicin per cm² of skin, such as about 500 to about 700 µg of capsaicin per cm² of skin, optionally administered to one or more areas of the skin of the foot) can show significant improvement in the vascular response of the skin and/or subcutaneous tissue to locally applied pressure, heat or certain chemicals, e.g. capsaicin. For example, they may show full or at least partial restoration of the normal ability of the vasculature to dilate in response to the application of local skin surface pressure, heat or chemicals. This may be measured experimentally, for example a human subject may have a temporary pressure, heat or a chemical applied to the skin surface and vasodilation and/or any resultant increase in blood flow maybe measured by any appropriate means including observation of skin reddening or measurement of blood flow by laser Doppler flowmetry.

### Patients

The uses of the present invention are provided for the prevention of diabetic ulcers, for example diabetic foot ulcers, in patients who are suffering from (painful and/or non-painful) diabetic peripheral neuropathy. Such patients are typically human subjects. A patient may be male or female. A patient may be of any age. Typically, a patient is an adult patient, for example a patient of from about 18 to about 80 years of age, from about 40 to about 80 years of age, or from about 60 to about 80 years of age. In certain preferred embodiments, a patient can be of from about 60 to about 80 years of age. A patient may be of any ethnic origin. A patient may have any body weight.

A patient may suffer from type 1 diabetes. A patient may suffer from type 2 diabetes. A patient may suffer from gestational diabetes.

A patient may have diabetes that is well controlled, or poorly controlled. A patient may have diabetes that is uncontrolled. For example, patients suffering from poorly controlled or uncontrolled diabetes may have an average blood sugar level over time (for example, over the course of 6 months, or over the course of 12 months) that is significantly higher (for example, 1 mmol/L or even higher) than the average blood sugar level over time of a healthy subject not suffering from diabetes. Patients suffering from well controlled, poorly controlled or uncontrolled diabetes may experience frequent infections. Patients suffering from well controlled, poorly controlled or uncontrolled diabetes may experience diabetic ulcers, for example diabetic foot ulcers. A patient may have already suffered from one or more diabetic ulcers, for example one or more diabetic foot ulcers. Having previously suffered from a diabetic foot ulcer may be used as a useful surrogate for ongoing susceptibility to further diabetic foot ulcers. A patient may be currently suffering from one or more diabetic ulcers, for example one or more diabetic foot ulcers. A patient may be currently suffering from one or more diabetic ulcers, for example one or more diabetic foot ulcers. Certain preferred embodiments of the invention concern diabetic patients liable to suffer from foot ulcers. A liability to suffer from diabetic foot ulcers can be indicated, for example, by the patient having suffered from one or more diabetic foot ulcers previously. A liability to suffer from diabetic foot ulcers on a particular foot can be indicated, for example, by the patient suffering from one or more diabetic foot ulcers on their other foot.

A patient may have been taking one or more medications for the treatment of diabetes. A patient may currently be taking one or more medications for the treatment of diabetes. A patient may have been taking one or more medications for the control of diabetes. A patient may currently be taking one or more medications for the control of diabetes. Exemplary medications include, but are not limited to: insulin; metformin; thiazolidinediones; gliptins; sodium-glucose cotransporter-2 inhibitors; sulfonylureas; and glucagon-like peptide agonists.

A patient may have an average haemoglobin Alc (HbAlc) level over three months of above 5.7 %; above 5.8 %; above 5.9 %; above 6.0 %; above 6.1 %; above 6.2 %; above 6.3 %; above 6.4 %; above 6.5 %; above 6.6 %; above 6.7%; above 6.8%; above 6.9%; above 7.0 %; above 7.1 %; above 7.2 %; above 7.3 %; above 7.4 %; above 7.5 %; above 7.6 %; above 7.7 %; above 7.8 %; above 7.9 %; above 8.0 %; above 8.1 %; above 8.2 %; above 8.3 %; above 8.4 %; above 8.5 %; above 8.6 %; above 8.7 %; above 8.8 %; above 8.9 %; or above 9.0 %.

The onset of (painful and/or non-painful) diabetic peripheral neuropathy in a patient may have occurred more than 1 year ago; more than 2 years ago; more than 3 years ago; more than 4 years ago; more than 5 years ago; more than 6 years ago; more than 7 years ago; more than 8 years ago; more than 9 years ago; more than 10 years ago; more than 11 years ago; more than 12 years ago; more than 13 years ago; more than 14 years ago; more than 15 years ago; more than 16 years ago; more than 17 years ago; more than 18 years ago; more than 19 years ago; more than 20 years ago; more than 21 years ago; more than 22 years ago; more than 23 years ago; more than 24 years ago; more than 25 years ago; more than 26 years ago; or more than 27 years ago.

The onset of pain associated with (painful and/or non-painful) diabetic peripheral neuropathy may have occurred in a patient, for example with pain and/or reduced sensitivity to temperature changes experienced in the region of one or more of the feet of the patient, may have occurred more than 1 year ago; more than 2 years ago; more than 3 years ago; more than 4 years ago; more than 5 years ago; more than 6 years ago; more than 7 years ago; more than 8 years ago; more than 9 years ago; more than 10 years ago; more than 11 years ago; more than 12 years ago; more than 13 years ago; more than 14 years ago; more than 15 years ago; more than 16 years ago; more than 17 years ago; more than 18 years ago; more than 19 years ago; more than 20 years ago; more than 21 years ago; more than 22 years ago; more than 23 years ago; more than 24 years ago; more than 25 years ago; more than 26 years ago; or more than 27 years ago.

The baseline pain score of a patient for spontaneous pain, for example spontaneous pain experienced in one or more of the feet of the patient, measured according to the Numerical Pain Rating Scale, may be more than 4, more than 5, more than 6, more than 7, or more than 8.

A patient may have been taking one or more medications for the management of pain associated with diabetic peripheral neuropathy (for example, pain experienced in one or more of the feet of the patient). A patient may currently be taking one or more medications for the management of pain associated with diabetic peripheral neuropathy (for example, pain experienced in one or more of the feet of the patient). Exemplary medications include, but are not limited to: pregabalin; duloxetine; amitriptyline; gabapentin; non-steroidal anti-inflammatory drugs; and opioids.

### Kit leaflet

Described herein and useful for understanding the disclosure, but not claimed, is a kit comprising a cutaneous patch and a leaflet, wherein the cutaneous patch contains capsaicin or a capsaicinoid or a TRPV1 agonist, and wherein the leaflet provides instructions for a method of preventing diabetic ulcers in a patient suffering from diabetic peripheral neuropathy, comprising administering the cutaneous patch to one or more areas of the skin of the patient.

The kit thus comprises a cutaneous patch and a leaflet. The leaflet provides instructions for a method of preventing diabetic ulcers, for example diabetic foot ulcers, in a patient suffering from (painful and/or non-painful) diabetic peripheral neuropathy, comprising administering the cutaneous patch to one or more areas of the skin of the patient.

The instructions in the leaflet can include instructions for carrying out a method which is directed to the prevention of diabetic ulcers, for example diabetic foot ulcers, in a patient suffering from diabetic peripheral neuropathy.

Such a kit may additionally comprise one or more items selected from cleansing gel, gauze, soap and personal protective equipment, such as gloves. It will be understood that a leaflet included in the kit may therefore provide instructions describing how to use one or more items selected from cleansing gel, gauze, soap and personal protective equipment.

The leaflet may provide instructions for administering capsaicin or a capsaicinoid or a TRPV1 agonist in a particular dosage using the cutaneous patch, for example a dosage described herein. The leaflet may provide instructions on how often to repeat treatment using the cutaneous patch, such as how treatment can be repeated at particular time intervals, for example at time intervals described herein.

The leaflet may provide instructions for administering capsaicin or a capsaicinoid or a TRPV1 agonist to a patient having one or more particular characteristics, as described herein. For example, the leaflet may provide instructions for administering capsaicin or a capsaicinoid or a TRPV1 agonist to a patient suffering from type 1 diabetes, or a patient suffering from type 2 diabetes, or a patient suffering from gestational diabetes. The leaflet may provide instructions for administering capsaicin or a capsaicinoid or a TRPV1 agonist to a patient suffering from poorly controlled diabetes, or uncontrolled diabetes. The leaflet may provide instructions for administering capsaicin or a capsaicinoid or a TRPV1 agonist to a patient who has been suffering from diabetes for more than a specified length of time, for example a length of time described herein. The leaflet may provide instructions for administering capsaicin or a capsaicinoid or a TRPV1 agonist to a patient who has already suffered from one or more diabetic ulcers. The leaflet may provide instructions for administering capsaicin or a capsaicinoid or a TRPV1 agonist to a patient who is currently suffering from one or more diabetic ulcers. The leaflet may provide instructions for administering capsaicin or a capsaicinoid or a TRPV1 agonist to a patient who is liable to suffer from foot ulcers. A liability to suffer from diabetic foot ulcers can be indicated, for example, by the patient having suffered from one or more diabetic foot ulcers previously. A liability to suffer from diabetic foot ulcers on a particular foot can be indicated, for example, by the patient suffering from one or more diabetic foot ulcers on their other foot.

It will be appreciated that the one or more areas of skin, for example one or more areas of the skin of the foot, to which the cutaneous patch is administered can differ from patient to patient, depending on their symptoms and signs of diabetic peripheral neuropathy and the extent of the diabetic peripheral neuropathy across the body surface. The one or more areas of skin, for example one or more areas of the skin of the foot, to which capsaicin or a capsaicinoid is administered can be one or more areas of skin, for example one or more areas of the skin of the foot, that exhibit symptoms and/or signs of diabetic peripheral neuropathy. Therefore, the instructions of the leaflet may include instructions on how to determine the one or more areas of skin, for example one or more areas of the skin of the foot, for exposure to the cutaneous patch, for example instructions describing how to determine the one or more areas of skin by touching different areas of skin (such as one or more areas of the skin of the foot) to determine whether each area of the skin has symptoms (for example, pain and/or reduced sensitivity to temperature changes). Additionally, or alternatively, the instructions may describe how to trace that information directly onto the skin of the patient, using for example an ink pen to mark the skin. Additionally, or alternatively, the instructions may describe how to obtain more sensitive, more accurate, or more objective information as to the extent of the peripheral neuropathy, by using neurophysiological testing, whereby a nerve conduction device is used to assess nerve fibre function.

The leaflet may provide instructions that warn the user about certain hazards associated with using the cutaneous patch. For example, the leaflet may state that hairs in the one or more areas of skin (for example one or more areas of the skin of the foot) should not normally be shaved (in order to avoid breaking the skin) but should instead be clipped, before gently washing the treatment area(s) with soap and water, then drying the area(s). The instructions may warn the user that, for reasons of safety, the cutaneous patch should not normally be administered to the skin of, or near, the eyes. Likewise, the instructions may warn the user that the cutaneous patch should not normally be administered to mucous membranes. The instructions may warn the user that the cutaneous patch should not normally be administered to the skin of the head, and in particular the cutaneous patch should not normally be administered to the skin of the face. The instructions may warn the user that the cutaneous patch should not normally be administered to broken, irritated or otherwise sensitive areas of skin, for example the skin of the anogenital regions of the body. The instructions may advise a first-time user to test the cutaneous patch initially on a small area of skin, and for a short period of time, before proceeding to use the cutaneous patch for treatment.

Whilst the present invention has been described and illustrated with reference to particular embodiments, it will be appreciated by those of ordinary skill in the art that the invention lends itself to many different variations not specifically illustrated herein. By way of example only, certain possible variations will now be described.

### Examples

### Example 1

### Materials and methods

### Study design

A single-centre longitudinal randomized study was conducted on the effects of capsaicin 8% patch treatment as licensed, in 50 patients with painful distal, symmetrical, sensorimotor polyneuropathy due to diabetes of at least 1-year duration. Study approval was obtained from the East of England, Cambridgeshire and Hertfordshire Research Ethics Committee (Ethics reference number: 17/EE/0498), the European Union Drug Regulating Authorities (EudraCT Number: 2017-004746-17), and registered in the ISRCTN registry (https://doi.org/10.1186/ISRCTN14254122). The study was monitored by the Joint Research Compliance Office, Imperial College London and Imperial College Healthcare NHS Trust, in accord with Good Clinical Practice (GCP) guidelines.

### Participants - subjects suffering from painful diabetic peripheral neuropathy

Patients enrolled in the study were randomly allocated with a 2 to 1 ratio to either receive 30-minute capsaicin 8% patch (Qutenza) application to both feet up to the distal calf, while continuing to take medication as part of the standard of care (Q+SOC group; n=25 completers at 3 months), or to receive standard of care alone (SOC group; n=12 completers at 3 months).

Patient demographics and characteristics are recorded in Table 1.

**Table 1**

| **Group** | **Capsaicin Patch + SOC** | **SOC** |
|---|---|---|
| Number of patients | n=25 | n=12 |

| **Age, Years** | | |
|---|---|---|
| Mean ± SD | 62 ± 11 | 64 ± 9.1 |
| Range | 40-83 | 51-84 |

| **Sex, n (%)** | | |
|---|---|---|
| Male | 12 (48%) | 5 (41%) |
| Female | 13 (52%) | 7 (59%) |

| **Type of Diabetes, n (%)** | | |
|---|---|---|
| Type I | 5 (20%) | 1 (8%) |
| Type II | 20 (80%) | 11 (92%) |

| **Duration of Diabetes, Mean, Years (Range; SD)** | | |
|---|---|---|
| | 18 (1-44; 11.3) | 15 (3-37; 11.1) |

| **Medications for Diabetes, n (%)** | | |
|---|---|---|
| Insulin alone | 6 (24%) | 2 (16%) |
| Metformin alone | 7 (28%) | 5 (42%) |
| Insulin + Metformin | 2 (8%) | 0 (0%) |
| Insulin or Metformin + thiazolidinediones / gliptins / (SGLT2) inhibitors / sulfonylureas / GLP agonist | 10 (40%) | 5 (42%) |

| **HbA1c % mean (Range), mmol/mol mean (Range)** | | |
|---|---|---|
| | 7.3 % (5.6 -9.0) 56 mmol/mol (38-75) | 6.8 % (5.5-8.7) 51.5 mmol/mol (37-72) |

| **Time elapsed since diagnosis of DPN, Mean; Years (Range; SD)** | | |
|---|---|---|
| | 7.3 (1-29; 6.8) | 9.0 (3-27; 6.6) |

| **Time elapsed since onset of pain associated with DPN, Mean; Years (Range; SD)** | | |
|---|---|---|
| | 7.6 (1-24; 5.9) | 8.5 (2-27; 6.9) |

| **Baseline Pain Score (NPRS) for spontaneous pain, Mean (Range; SD)** | | |
|---|---|---|
| | 7.1 (4.4-9.5; 1.6) | 8.1 (4.8-10; 1.6) |

| **Medications for Pain, n (%)** | | |
|---|---|---|
| Pregabalin | 10 (40%) | 8 (67%) |
| Duloxetine | 6 (24%) | 4 (33%) |
| Amitriptyline | 4 (16%) | 1 (8%) |
| Gabapentin | 2 (8%) | 1 (8%) |
| NSAIDs | 5 (20%) | 3 (25%) |
| Opioids | 5 (20%) | 6 (50%) |

| **NCS (mean ± SEM; Range)** | | |
|---|---|---|
| Peroneal motor action potential amplitude | 3.6 ± 0.7 (0.0-15.2) µV | 1.8 ± 0.7 (0.0-6.4) µV |
| Peroneal conduction velocity | 38.7 ± 3.4 (0.0-53) m/s | 31.9 ± 5.8 (0.0-55.6) m/s |
| Sural sensory action potential amplitude | 6.9 ± 1.2 (0.0-21.6) µV | 1.8 ± 0.6 (0.0-6.0) µV |
| Sural nerve conduction velocity | 41.8 ± 4.9 (0.0-70.2) m/s | 27.0 ± 7.1 (0.0-61.2) m/s |

| | | |
|---|---|---|
| ***Abbreviations*:** SOC, Standard of Care; SD, Standard Deviation; SGTL2, Sodium-glucose Cotransporter-2; GLP, Glucagon-like peptide; NSAIDs, Nonsteroidal anti-inflammatory drugs; NPRS, Numerical Pain Rating Scale; DPN, Diabetic Peripheral Neuropathy. | | |

Patients were given a trial diary to complete starting on the day of screening and continuing for the next 7 days. The diary collected pain rating scores twice daily (ranging from 0 to 10). An 11-point numerical rating scale (NPRS) with the 0-anchor point being "no pain" and the 10-anchor point being "pain as bad as you can imagine", to describe "pain on average in the last 24 hours" was used for both spontaneous and evoked pain. After completing this diary for 7 days, the result of their NPRS was used to determine eligibility for the study. Only patients with average pain intensity equal to or greater than 4 /10 for spontaneous pain were eligible to participate further in the study, and were advised to continue completing the diary daily for the entire duration of the study until the end-of-study follow up visit (Figure 1). Application of Capsaicin 8% Patch was performed within 1 month from the baseline visit. All patients were invited for follow-up assessments 3-monthly after Capsaicin 8% Patch application.

Pain was described mostly as burning pain (64%) and less commonly as cramping pain (6%), sharp pain (11%), shooting pain (6%); throbbing/ stabbing pain (6%) or aching pain (3%).

29 out of 37 subjects were taking treatments for neuropathic pain at the start of the study (Pregabalin, Duloxetine, Amitriptyline, Gabapentin, NSAIDs, opioids, or a combination of these), but not topical treatments. The mean (SEM) of the Numeric Pain Rating Score (NPRS) at the first visit was 7.1 (0.31) for Q+SOC group and 8.1 (0.46) for SOC group. The mean ± SEM of the SF-McGill Questionnaire overall pain score at the first visit was 106(9.5) for Q+SOC group and 126 (15.2) for SOC group.

### Clinical examination

Clinical examination and tests confirmed that patients had a predominantly sensory, length-dependent polyneuropathy. Distal weakness (lower limb) was present in 1 out of 36 patients at Medical Research Council grade < 4/5. Neurological deficits were recorded using the Neuropathy Impairment Score Lower Limbs (NIS-LL) (Bril, Eur Neurol. 1999, 41 Suppl 1:8-13) which is a summed score including of muscle power, reflex loss (maximum score 88, indicating severe neuropathy). The mean ± SEM NIS-LL at the baseline visit was 10.7 (1.1) for Q+SOC group and 12 (0.8) or SOC group.

### Assessment of neuropathy

Clinical examination and tests were performed in both lower and upper limbs, and the right lower limb values were used for analyses. Nerve conduction studies were performed by a senior consultant neurophysiologist. Symptoms were recorded using the Short-Form McGill Pain Questionnaire (Melzack, Pain. 1987, 30:191-197) with maximum score 220, indicating severe symptoms.

### Short-Form McGill Pain Questionnaire (SF-MPQ-2)

Participants completed the SF-MPQ-2 by rating the extent to which they experienced each of 22 pain descriptors in the past week. The SF-MPQ-2 is composed of 4 summary scales: 1) continuous descriptors (throbbing pain, cramping pain, gnawing pain, aching pain, heavy pain, and tender); 2) intermittent descriptors (shooting pain, stabbing pain, sharp pain, splitting pain, electric-shock pain, and piercing); 3) neuropathic descriptors (hot-burning pain, cold freezing pain, pain caused by light touch, itching, tingling or pins and needles, and numbness); and 4) affective descriptors (tiring-exhausting, sickening, fearful, and punishing-cruel). A total pain score was computed by averaging participant ratings across all questions, while scale pain scores were derived from averaging ratings to questions that comprise the respective scales.

### Patient Global Impression of Change

Patients were asked to complete the Clinical Global Impression of Change; this was completed at the baseline visit and three-monthly visits. The Questionnaire is composed of a 7-point scale, enabling the patient to indicate no change, improvement or worsening of their condition.

### Quantitative sensory testing

For quantitative sensory testing (QST), thresholds for light touch were measured using Semmes-Weinstein hairs (made by A. Ainsworth, University College London, UK); No. 1 (0.0174 g) to No. 20 (263.0 g). The hair with the lowest force reliably detected by the patient on the dorsum of the toe was recorded. Values > 0.0479 g were considered abnormal (Atherton et al., BMC Neurol. 2007, 7:21).

Vibration perception thresholds were measured using a biothesiometer (Biomedical Instrument Company, Newbury, OH, USA) placed on the metatarsophalangeal joint of the big toe. Three ascending and three descending trials were carried out, and the mean value obtained. Values >12 V were considered abnormal (Coppini et al., J. Clin. Neurosci. 2001, 8:520-524).

Thermal perception thresholds were performed as described (Anand et al., Nat. Med. 1996, 2:703-707; Wellmer et al., J Peripher Nerv Syst. 2001, 6:204-210) using the TSA II-NeuroSensory Analyzer (Medoc, Ramat Yishai, Israel). A 30 mm x 30 mm thermode was used and thermal thresholds determined in the soles of the feet (under the instep), for warm perception, cool perception, heat pain and cold pain from a baseline temperature of 32°C, with a change in temperature of 1oC /s. The mean of three consecutive tests for each modality was recorded. Values >6.4 °C for warm sensation, >2.3°C for cool sensation and >10.4°C for heat pain, were considered abnormal (Anand et al., Nat. Med. 1996, 2:703-707; Atherton et al., BMC Neurol. 2007, 7:21; Wellmer et al., J Peripher Nerv Syst. 2001, 6:204-210).

### Nerve conduction studies

Nerve conduction studies of the common peroneal (including F wave studies) and sural nerves in the right leg were performed with a Medtronic Keypoint device (Medtronic, Minneapolis, MN, USA). Nerve conduction studies were performed once at the baseline visit for all patients. Sural antidromic sensory action potentials of <5 µV amplitude and <40 m/s conduction velocity, common peroneal nerve (compound muscle action potential from extensor digitorum brevis) values <3 mV amplitude, <40 m/s conduction velocity, and F-wave latency >60 msec were considered abnormal (Atherton et al., BMC Neurol. 2007, 7:21, Narayanaswamy et al., J. Clin. Neurosci. 2012, 19:1490-1496).

### Calf skin biopsy and immunohistochemistry

Two 3.5-mm diameter skin punch biopsies were collected under local anaesthesia from the distal lateral calf of patients at baseline (visit 1) before capsaicin 8% patch (Qutenza) application, and repeated 3 months after the initial visit or treatment, within the area of capsaicin 8% patch treatment. Skin biopsies collected from age and gender-matched healthy volunteers were analyzed alongside the patient's biopsies, as controls.

The immunohistochemical methods and antibodies used have been reported previously (Ragé et al., Clin Neurophysiol. 2010, 121(8):1256-1266; Gopinath et al., BMC Women's Health. 2005, 5(1):2; Facer et al., Brain. 1998, 121(Pt.12):2239-2247; Anand et al., Pain Manag. 2019, 9:521-533). One of the two skin biopsies was snap frozen and stored at -70°C, and the other immersed in fixative (modified Zamboni's fluid - 2% formalin; 0.01 M phosphate buffer; 15% saturated picric acid (pH 7.2), then washed in phosphate buffered saline (PBS; 0.1 M phosphate; 0.9% w/v saline; pH 7.3) containing 15% w/v sucrose for an hour, before snap freezing in optimum cutting tissue embedding medium (Tissue-Tek OCT, RA Lamb Ltd, Eastbourne, U.K.). Frozen sections (15µm thickness) were collected onto poly-L-lysine (Sigma, Poole, UK) coated glass slides and post-fixed in freshly prepared, 4% w/v paraformaldehyde in 0.15M phosphate buffered saline (PBS) for 30 min. Sections of pre-fixed tissue were collected in the same way and allowed to air dry for markers. Endogenous peroxidase was blocked by incubation in methanol containing 0.3% w/v hydrogen peroxide for 30 minutes for both post- and pre-fixed sections. After rehydration, appropriately processed sections were incubated overnight with primary antibodies.

The antibodies were to the structural nerve marker PGP 9.5 (Rabbit, RA95/06, 1:40,000; Ultraclone, Isle of Wight, UK), the nerve regeneration marker, growth associated protein GAP-43 (G9264, Mouse, 7B10, 1:80,000; Sigma, Poole, UK), von Willebrand factor vWF (Rabbit, 1:10,000; Novocastra Laboratories, Milton Keynes, UK). 50-µm sections were also studied with PGP 9.5 antibody.

Briefly, fixed sections were floated onto PBS in 12-well plates, dehydrated with alcohol/hydrogen peroxide solution for 30 min, washed with PBS and incubated with PGP 9.5 overnight, washed and incubated with the second antibody for 1 h, and then washed and incubated with ABC as above. After washing, the nickel developer solution was added, and staining allowed to develop. The reaction was stopped by adding 0.1 M sodium acetate pH 6.0, washed again in PBS, counterstained and free floated onto PPL slides, allowed to dry and incubated in xylene, and finally mounted using DPX mountant.

Nerve fibres were counted along the length of four nonconsecutive sections. The length of epithelium in each counted section was measured using computerized microscopy software (Olympus ANALYSIS 5.0 Soft, Olympus UK, Southend, Essex, UK) and results expressed as fibres/mm length of the section. Sub-epidermal nerve immune-reactivity obtained as a percentage (% area) measured by image analysis where digital photomicrographs were captured via video link to an Olympus BX50 microscope. The grey-shade detection threshold was set at a constant level to allow detection of positive immuno-staining and the area of highlighted immuno-reactivity was expressed as a percentage (% area) of the field scanned. Images were captured (x40 objective magnification) along the entire length, and the mean values were used for statistical analysis. Quantification was performed by two independent blinded observers, and there was no significant difference between observers. Validation of these methods, including vs. PGP9.5 IENF in 50µm vs. 15 µm thickness sections, have been published previously (Van Acker et al., BMC Res Notes 9, 280 (2016**),** Anand et al, J. Pain Res. 2017, 10:1623-1634). The GAP 43 IENFs were sparse and so only SENFs were analyzed (as in Narayanaswamy et al., J. Clin. Neurosci. 2012, 19:1490-1496).

### Statistical analysis

Data were analyzed using GraphPad Prism version 5.0 for Windows (GraphPad Prism Software, San Diego, CA, USA). The statistical tests used were paired two-tailed Mann-Whitney test, Student's t-test, two-way ANOVA analysis, and Spearman's correlation test. For all statistical tests, p values <0.05 were considered significant.

### Results

There was no significant difference in the NIS-LL score before and after the treatment of capsaicin 8% patch (Q+SOC group). No statistically significant changes in NIS-LL were observed in SOC alone group.

### Nerve Conduction Study

Most patients (86%; n=32) had at least one abnormality on nerve conduction study at the first visit; 62% (n=23) had both motor and sensory abnormalities. F-waves were absent or not recorded in 37% (n=14) patients. The peroneal motor action potential mean ± SEM (range) was 3.6 ± 0.7 (0.0 - 15.2) µV and 1.8 ± 0.7 (0.0 - 6.4) µV in Q+SOC group and SOC group respectively. The peroneal conduction velocity mean ± SEM (range) was 38.7 ± 3.4 (0.0 - 53) m/s in Q+SOC group and 31.9 ± 5.8 (0.0 - 55.6) m/s in SOC group. The peroneal conduction velocity was absent in 4 patients in the Q+SOC group and 4 patients in the SOC group. Sural sensory action potential was 6.9 ± 1.2 (0.0 - 21.6) µV and 1.8 ± 0.6 (0.0 - 6.0) µV in Q+SOC group and SOC group respectively. The sural nerve velocity was 41.8 ± 4.9 (0.0 - 70.2) m/s in Q+SOC group and 27.0 ± 7.1 (0.0 - 61.2) m/s in SOC group. The sural nerve velocity was absent in 6 patients in Q+SOC group and 5 patients in SOC group (Table 1). Normal values for nerve conduction studies have been presented previously (Narayanaswamy et al., J. Clin. Neurosci. 2012, 19:1490-1496).

### Pain Scores and Questionnaires

There was a significant reduction in the average daily NPRS three months after capsaicin 8% patch application (baseline week vs. week 12 after patch application; Figure 2) for the Q+SOC group, mean difference of NPRS between baseline and 3 months follow-up -1.87, ***p=0.0001; Figure 3). The change in average pain score at the 3-month follow-up visit for the SOC group was not significant (mean difference of NPRS between baseline and 3 months follow-up -0.58, p=0.11).

At the follow-up visit 3 months after the treatment for Q+SOC group, SF-MPQ-2 showed a significant reduction in the overall pain score (-31.1; **p=0.002), intermittent descriptors of pain (-9.4; **p=0.005) , neuropathic descriptors (-5.9; *p=0.02) and affective descriptors of pain (-9.4;**p=0.001). There was no significant difference in these scores in the SOC group. Overall SF-MPQ-2 pain score was positively correlated with the improvement of warm thresholds (*p=0.04). Patient's Global Impression of Change (PGIC) showed significant improvement in the Q+SOC group (*p=0.01), but not in the SOC group (Table 2).

**Table 2**

| *Results at baseline and at 3 month follow-up visit for patients in the Capsaicin 8% Patch plus Standard of Care (Q+SOC) group, and Standard of Care alone group (SOC), for Spontaneous pain (NPRS; paired t-test), Short Form McGill Pain Questionnaire (SF-McGill; Mann-Whitney test), Patient Global Impression of Change (PGIC; paired t-test, baseline PGIC was change from screening to baseline visit), and Quantitative Sensory Testing (QST; paired t-test).* | | | |
|---|---|---|---|
| **Numerical Pain Rating Scale (NPRS) for Spontaneous pain [Mean ± SEM]** | | | |
| **Patients treated with Capsaicin 8% Patch (Q+SOC)** | **Baseline** (Pre-treatment) | **3 Month FU** (Post-treatment) | **p-value** |
| | 7.1 ± 0.31 | 5.3 ± 0.55 | ***p=0.0001 |
| **Patients not treated with Capsaicin 8% Patch (SOC)** | **Baseline** | **3 Month FU** | **p-value** |
| | 8.1 ± 0.46 | 7.5 ± **0.51** | P=0.11 |

| **Short Form McGill pain Questionnaire (SF-MPQ) [Mean ± SEM]** | | | |
|---|---|---|---|
| **Patients treated with Capsaicin 8% Patch (Q+SOC)** | **Baseline** (Pre-treatment) | **3 Month FU** (Post-treatment) | **p-value** |
| Continuous pain | 27.2 ± 2.9 | 21.1 ± 3.1 | p=0.07 ns |
| Intermittent pain | 28.3 ± 3.1 | 18.9 ± 3.3 | **p=0.005 |
| Affective pain | 20.4 ± 2.4 | 10.9 ± 2.4 | **p=0.001 |
| Neuropathic pain | 31.8 ± 2.7 | 25.9 ± 2.9 | *p=0.02 |
| Overall pain | 106.4 ± 9.5 | 75.2 ± 10.9 | **p=0.002 |

| **Patients not treated with Capsaicin 8% Patch (SOC)** | **Baseline** | **3 Month FU** | **p-value** |
|---|---|---|---|
| Continuous pain | 35.5 ± 4.5 | 34.1 ± 5.8 | p=0.75 |
| Intermittent pain | 32.5 ± 5.0 | 34.8 ± 5.1 | p=0.62 |
| Affective pain | 23.0 ± 3.3 | 19.0 ± 4.3 | p=0.17 |
| Neuropathic pain | 35.0 ± 5.0 | 37.5 ± 5.5 | p=0.44 |
| Overall pain | 126.0 ± 15.2 | 124.0 ± 18.0 | p=0.90 |

| **Patient Global Impression of Change (PGIC) [Mean ± SEM]** | | | |
|---|---|---|---|
| **Patients treated with Capsaicin 8% Patch (Q+SOC)** | **Baseline** (Pre-treatment) | **3 Month FU** (Post-treatment) | **p-value** |
| | 4.0 ± 0.0 | 3.0 ± 0.3 | *p=0.007 |
| **Patients not treated with Capsaicin 8% Patch (SOC)** | **Baseline** | **3 Month FU** | **p-value** |
| | 3.7 ± 0.2 | 4.0 ± **0.2** | p=0.11 |

| **Quantitative Sensory Testing (QST) [Mean ± SEM]** | | | |
|---|---|---|---|
| **Patients treated with Capsaicin 8% Patch ( Q+SOC)** | **Baseline** (Pre-treatment) | **3 Month FU** (Post-treatment) | **p-value** |
| Cool Threshold (°C) | 8.9 ± 0.8 | 8.4 ± 0.7 | p=0.61 |
| Warm Threshold (°C) | 13.1 ± 0.8 | 11.4 ± 0.9 | *p=0.02 |
| Cold Pain Threshold (°C) | 20.2 ± 1.5 | 18.7 ± 1.5 | p=0.29 |
| Heat Pain Threshold (°C) | 16.6 ± 0.4 | 16.2 ± 0.4 | p=0.41 |
| Monofilament Threshold (V) | 12.1 ± 10.5 | 11.7 ± 10.5 | p=0.29 |
| Vibration Threshold (g) | 33.1 ± 2.7 | 32.7 ± 2.7 | p=0.69 |

| **Patients not treated with Capsaicin 8% Patch (SOC)** | **Baseline** | **3 Month FU** | **p-value** |
|---|---|---|---|
| Cool Threshold (°C) | 15.1 ± 3.0 | 13.8 ± 2.1 | p=0.41 |
| Warm Threshold (°C) | 16.7 ± 0.6 | 16.3 ± 0.6 | p=0.40 |
| Cold Pain Threshold (°C) | 28.7 ± 1.6 | 26.0 ± 2.1 | p=0.16 |
| Heat Pain Threshold (°C) | 17.9 ± 0.1 | 16.7 ± 0.7 | p=0.14 |
| Monofilament Threshold (V) | 44.5 ± 29.4 | 45.1 ± 29.3 | p=0.33 |
| Vibration Threshold (g) | 38.2 ± 3.7 | 41.2 ± 2.9 | p=0.21 |

| **Neuropathy Impairment Score Lower Limbs (NIS-LL) [Mean ± SEM]** | | | |
|---|---|---|---|
| **Patients treated with Capsaicin 8% Patch (Q+SOC)** | **Baseline** (Pre-treatment) | **3 Month FU** (Post-treatment) | **p-value** |
| | 10.7 ± 1.1 | 10.5 ± 1.0 | p=0.63 |
| **Patients not treated with Capsaicin 8% Patch (SOC)** | **Baseline** | **3 Month FU** | **p-value** |
| | 12.0 ± 0.8 | 12.1 ± 1.1 | p=0.88 |

| | | | |
|---|---|---|---|
| ***Abbreviations*:** SEM, Standard error of the mean; NPRS, Numerical Pain Rating Scale; PGIC, Patient Global Impression of Change; QST, Quantitative Sensory Testing; NIS-LL, Neuropathy Impairment Score Lower Limbs; V, Volts; g, Grams, FU follow up. | | | |

### Quantitative Sensory Testing

There were no significant changes in the touch, vibration, cool or heat pain thresholds, in Q+SOC or SOC groups. The Q+SOC group showed a statistically significant improvement of warm perception threshold (mean ± SEM (range) at baseline 13.1 (0.8) and 3 months after Capsaicin 8% patch application (mean ± SEM (range) 11.4 (0.9); *p=0.02; Figure 4). There was no change in thresholds in the SOC group (Table 2).

### Immunohistochemistry

### PGP9.5 IENFs

Calf skin biopsies in the Q + SOC group (n=25) showed a statistically significant increase of PGP9.5 marker for IENFs in 15 µm sections (***p=0.0005; Figure 5) and 50-µm sections (***p=0.0002, Figure 6). Comparison between the control group and patients with painful diabetic neuropathy for PGP9.5 IENFs before and after patch application showed statistically significant lower density in both 15 µm and 50 µm sections (Figure 7 and Figure 8). No statistically significant changes were observed in the SOC group (n=12) between baseline and biopsies after 3 months (Tables 3a and 3b).

**Table 3a**

| *Skin biopsy markers assessed at baseline and at 3 months follow-up visit in Q+SOC group, for PGP 9.5 in 15µm and 50µm thick sections.* | | | | | | |
|---|---|---|---|---|---|---|
| **Capsaicin 8% Patch (Qutenza) + SOC** | | | | | | |
| **PGP IENFs** | 15µm | | **Mean** | **SD** | **SEM** | **p value; n=25** |
| | | Baseline | 0.705 | 1.148 | 0.234 | ***p=0.0005 |
| | | 3 months FU | 1.725 | 1.643 | 0.335 | |
| | 50 µm | | **Mean** | **SD** | **SEM** | **p value; n=25** |
| | | Baseline | 1.507 | 2.225 | 0.454 | ***p=0.0002 |
| | | 3 months FU | 3.469 | 3.283 | 0.670 | |
| **PGP SENFs** | 15 µm | | **Mean** | **SD** | **SEM** | **p value; n=25** |
| | | Baseline | 0.549 | 0.278 | 0.057 | ****p=0.003** |
| | | 3 months FU | 0.790 | 0.374 | 0.076 | |
| | 50 µm | | **Mean** | **SD** | **SEM** | **p value; n=25** |
| | | Baseline | 1.060 | 0.529 | 0.108 | ***p=0.0001 |
| | | 3 months FU | 1.628 | 0.861 | 0.176 | |
| **GAP SENFs** | | | **Mean** | **SD** | **SEM** | **p value; n=25** |
| | | Baseline | 0.262 | 0.270 | 0.054 | **p=0.003 |
| | | 3 months FU | 0.462 | 0.350 | 0.070 | |
| **vWF** | | | **Mean** | **SD** | **SEM** | **p value; n=25** |
| | | Baseline | 4.178 | 1.160 | 0.232 | p=0.18 |
| | | 3 months FU | 4.546 | 1.943 | 0.389 | |

| | | | | | | |
|---|---|---|---|---|---|---|
| ***Abbreviations**:* IENFs, Intra-epidermal Nerve Fibres density (fibres/mm); SENFs (% Area), Sub-epidermal Nerve Fibres density; PGP 9.5 , Pan-neuronal marker protein gene product 9.5; GAP 43, Growth-associated protein 43; vWF, von Willebrand Factor. | | | | | | |

**Table 3b**

| *Skin biopsy markers assessed at baseline and at 3 months follow-up visit in SOC alone group, for PGP 9.5 in 15µm and 50µm thick sections.* | | | | | | |
|---|---|---|---|---|---|---|
| **SOC** | | | | | | |
| **PGP IENFs** | 15µm | | **Mean** | **SD** | **SEM** | **p value; n=12** |
| | | Baseline | 0.025 | 0.087 | 0.025 | p=0.62 |
| | | 3 months FU | 0.058 | 0.202 | 0.058 | |
| | 50 µm | | **Mean** | **SD** | **SEM** | **p value; n=12** |
| | | Baseline | 0.150 | 0.350 | 0.101 | p=0.57 |
| | | 3 months FU | 0.244 | 0.721 | 0.208 | |
| **PGP SENFs** | 15 µm | | **Mean** | **SD** | **SEM** | **p value; n=12** |
| | | Baseline | 0.239 | 0.136 | 0.039 | p=0.95 |
| | | 3 months FU | 0.238 | 0.158 | 0.046 | |
| | 50 µm | | **Mean** | **SD** | **SEM** | **p value; n=12** |
| | | Baseline | 0.431 | 0.173 | 0.050 | p=0.85 |
| | | 3 months FU | 0.444 | 0.239 | 0.069 | |
| **GAP SENFs** | | | **Mean** | **SD** | **SEM** | p **value; n=12** |
| | | Baseline | 0.110 | 0.111 | 0.032 | *p=0.04 |
| | | 3 months FU | 0.060 | 0.085 | 0.024 | |
| **vWF** | | | **Mean** | **SD** | **SEM** | **p value; n=12** |
| | | Baseline | 5.158 | 1.635 | 0.472 | p=0.7 |
| | | 3 months FU | 5.278 | 1.282 | 0.370 | |

| | | | | | | |
|---|---|---|---|---|---|---|
| ***Abbreviations**:* IENFs, Intra-epidermal Nerve Fibres density (fibres/mm); SENFs (% Area), Sub-epidermal Nerve Fibres density; PGP 9.5 , Pan-neuronal marker protein gene product 9.5; GAP 43, Growth-associated protein 43; vWF, von Willebrand Factor. | | | | | | |

### PGP9. 5 SENFs

At 3 months after treatment, Q + SOC group showed a statistically significant increase of PGP9.5 SENFs in 15 µm sections (**p=0.003; Figure 9), and 50-µm calf skin sections (***p=0.0001; Figure 10). PGP9.5 IENFs fibres before and after capsaicin 8% patch application showed statistically significant differences between controls (healthy volunteers) and the Q+SOC group before but not after capsaicin 8% patch application, for both 15 µm and 50 µm thick sections (Figure 11 and Figure 12). No statistically significant changes were observed in the SOC group (Tables 3a and 3b).

### GAP43

The Q+SOC group showed a significant increase of GAP43 for SENFs compared to baseline biopsies (**p=0.003; Figure 13). Comparison between controls and Q+SOC group for GAP43 SENFs showed no significant difference before capsaicin 8% patch application, but these were significantly higher after patch application in the Q+SOC group (*p=0.02; Figure 14). A significant decrease was observed over 3 months in the SOC group; *p=0.04 (Tables 3a and 3b).

### von Willebrand Factor (vWF)

No changes in von Willebrand Factor (vWF) was observed in the Q+SOC group after treatment (Figure 15). Comparison between controls and patients with painful diabetic neuropathy before and after capsaicin 8% patch (Qutenza) application showed significantly higher levels in PDPN (Figure 16), as we have reported previously in a different cohort of PDPN/DPN patients (Shillo et al., Diabet. Med. 2017; 34(S1):64). No significant changes were observed in the SOC group (Tables 3a and 3b).

### Case report

The first participant in the study, who was randomized to the capsaicin 8% patch (Qutenza) treatment plus Standard of Care group, is the subject of this illustrative case report. The patient had to the option to continue with 3-monthly assessments over a year following the baseline visit.

### History

A 65 year old patient had a 14-year history of type 2 diabetes, well managed with Insulin and Metformin. When first seen in our Neuropathy clinic 3 years prior to her enrolment in our present study, at the request of our Diabetes Clinic colleagues, she gave a 4-year history then of persistent pain in lower limbs including feet. The pain had been attributed to diabetic neuropathy following investigations, including imaging of the spine and assessments by the Vascular Surgery team. She also had other medical problems (glaucoma and hypertension). When seen initially in she reported sharp pains and tingling in both lower limbs with a Numerical Pain Rating Scale (NPRS) score of up to 9/10. Clinical examination showed no significant weakness, absent deep tendon reflexes at the ankle, and down-going plantar responses. Pin prick and joint position sense were preserved in fingers and toes. Quantitative sensory testing (QST) showed thermal thresholds were significantly elevated for warm and marginally elevated for cold perception in both feet, but were normal in the hands. Vibration perception thresholds were normal in the hands and feet. Pregabalin was suggested as a treatment for her pain, in increasing doses, which provided partial pain relief. Duloxetine was contra-indicated as she had glaucoma.

### Baseline visit for Capsaicin 8% Patch (Qutenza) clinical trial

The patient was invited to take part in this study as her neuropathic pain symptoms persisted. At the baseline visit, the pain was described as burning and shooting, with a NPRS of 7/10. She was taking Pregabalin 450 mg daily in divided does. Clinical examination now showed reduced pinprick sensation to mid-palms in both hands and to mid-shins in both legs, but was otherwise unchanged. QST now showed significantly elevated thermal thresholds for warm perception in both feet, and marginal elevation in both hands. Cool perception thresholds were elevated to a lesser extent in both feet, but within normal limits in both hands. Monofilament and vibration thresholds were normal in the hands and feet. Nerve conduction study was within the normal range. Skin biopsy from the calf showed intra-epidermal nerve fibres (IENF) were markedly reduced at 0.4 fibres/mm for the pan-neuronal marker PGP9.5. The sub-epidermal nerve fibres (SENF) were also reduced, at 0.42.

### Capsaicin 8% Patch (Qutenza) treatment and 3-monthly follow-up visits

Capsaicin 8% Patch was applied in both feet up to the distal calf above the skin biopsy site for 30 minutes, after the skin biopsy had healed; the procedure was uneventful, and no adverse effects were recorded. Cooling packs were applied during the Capsaicin 8% Patch (Qutenza) application to ameliorate any symptoms associated with patch application. The application of the capsaicin patch was followed by further assessments 3, 6, 9 and 12 months after the treatment.

At the first follow-up visit, after 3 months, the spontaneous pain score in the legs had decreased from 7/10 at baseline to 1.2/10 (Figure 17); she reported the pain in the feet had completely disappeared 2 weeks after the treatment (0/10), at the site of Capsaicin 8% Patch (Qutenza) application. Cold and touch-evoked pain symptoms were similarly improved in the NPRS. Clinical examination and quantitative sensory testing did not show differences compared to the previous findings. Skin biopsy from the calf, performed within the Capsaicin 8% Patch (Qutenza) application site, showed intra-epidermal nerve fibres (IENF) for PGP9.5 at 3.2 fibres/mm and sub-epidermal nerve fibres (SENF) at 0.9 fibres/mm for PGP9.5 and other nerve markers (Figure 18). HbA1c was similar (51 mmol/mol) to the baseline value (46 mmol/mol).

As per standard clinical practice and criterion for the study (pain score NPRS <4/10), capsaicin 8% patch treatment was not repeated, and a second follow-up after 6 months was arranged. At the second follow up visit the pain score was reported as 3.3/10. Clinical examination had not changed. Skin biopsy showed intra-epidermal nerve fibres (IENF; 50 µm sections) for PGP9.5 at 5.90 fibres/mm (Figure 18) and sub-epidermal nerve fibres (SENF; 50 µm sections) at 2.78 fibres/mm. After 9 months the pain score was reported as 2.9/10. The clinical examination did not differ, and the skin biopsy showed intra-epidermal nerve fibres (IENF; 50 µm sections) for PGP9.5 at 9.30 fibres/mm (Figure 18) and sub-epidermal nerve fibres (SENF; 50 µm sections) at 1.60 fibres/mm. After 12 months the pain score was reported as 3.1/10. The clinical examination did not differ, and the skin biopsy showed intra-epidermal nerve fibres (IENF; 50 µm sections) for PGP9.5 at 7.34 fibres/mm (Figure 18) and sub-epidermal nerve fibres (SENF; 50 µm sections) at 1.9 fibres/mm.

### Conclusions

This case exemplifies the potential of Capsaicin 8% Patch (Qutenza) for long-term pain relief with regeneration and restoration of cutaneous nerve fibres in chronic diabetic peripheral neuropathy. We have not observed such a marked significant effect of any pain or neuropathy treatment previously in diabetic peripheral neuropathy patients.

### Discussion

Reduction of spontaneous (ongoing) pain in the Q+SOC group was significant, and showed a similar time-course, effect-size and responder rates to previous studies (e.g. Martini et al., J Pain Res. 2012, 5:51-59). As expected, pain relief was significant from week 3 onwards after Capsaicin 8% Patch (Qutenza) treatment, with some return towards baseline at 3 months. The first subject who completed the study showed pain relief persisting for 12 months after a single 30-minute Capsaicin 8% Patch application (as reported for pain relief in around 1/3 subjects, persisting at 3 months post-treatment, by Martini et al., J Pain Res. 2012, 5:51-59, though they did not assess skin biopsies). Our subject illustrates the potential of capsaicin 8% patch (Qutenza) for pain relief with disease modification i.e. regeneration and restoration of cutaneous nerve fibres in skin biopsies, collected 3-monthly from treated region.

This increase of nerve fibres was also shown overall in the Q+SOC group at 3 months after capsaicin 8% patch (Qutenza) treatment, for both intra-epidermal (IENFs) and sub-epidermal nerve fibres (SENFs) with the pan-neuronal marker PGP9.5; further, the SENFs were no longer significantly lower than in control skin biopsies (collected from healthy volunteers). Sub-epidermal nerve fibres (SENF) assessed with GAP 43, a selective marker of regenerating nerve fibres, were also significantly higher 3 months after treatment, and indeed higher than controls. In contrast, these were decreased in the SOC group after 3 months, in comparison with their baseline biopsies. Thermal thresholds for warm perception in the Q+SOC group were significantly improved 3 months after treatment, which correlated significantly with the improvement in the overall pain score in the Short-Form McGill Pain Questionnaire.

No such effect on sensory thresholds or nerve fibre density has previously been observed with any pain or neuropathy treatment in DPN patients. The patients randomized to SOC alone in the present study showed no significant changes in pain scores, skin biopsies or sensory perception thresholds at the 3-month follow-up visit.

Progressive loss of nerve fibres in skin biopsies collected from 29 patients who had diabetes for 18 years and reported DPN symptoms for a period of 4 years has been reported previously. They had assessments on two visits, with a 6-month interval (Narayanaswamy et al., J. Clin. Neurosci. 2012, 19:1490-1496). In that study, the annual rate ± SEM of IENFs loss was calculated to be 3.76 ± 1.46 fibres/mm for PGP9.5, and 3.13 ± 0.58 fibres/mm for TRPV1.

The time-course of nerve regeneration following high-dose topical capsaicin in human volunteers has been reported previously (Ragé et al., Clin Neurophysiol. 2010, 121(8):1256-1266) - a range of nerve markers were studied, including PGP9.5, GAP 43 and TRPV1 (the heat and capsaicin receptor). Experimental studies with low-concentration topical capsaicin have investigated intra-epidermal PGP9.5 nerve fibre regeneration (following initial degeneration) in the skin of individuals with diabetes, and have reported the degree and rate of regeneration seen in diabetic patients was lower when compared to healthy controls. One study showed the effect of topical application of an occlusive bandage containing 1.8 g of 0.1% capsaicin cream for two consecutive 24 h periods in healthy volunteers and subjects with DM with or without PDN (Polydefkis et al., Brain 2004, 127:1606-1615). Follow-up skin biopsies showed the mean rate of re-innervation in subjects with diabetes was reduced compared with control subjects, and was even slower in those with neuropathy compared with those without neuropathy. However, while no differences were found between patients with type I versus type II diabetes, another study showed a slower regeneration rate in type 2 DM compared to type 1 DM, despite the shorter duration of the DM (Khoshnoodi et al., Ann Clin Transl Neurol. 2019, 6:2088-2096). The depth and degree of "defunctionalization" of cutaneous nerve fibres (see Anand and Bley, Br. J. Anaesth. 2011, 107:490-502) may have been different in these studies compared to the effect of Capsaicin 8% Patch, and they did not assess sub-epidermal or regenerating nerve fibres specifically (the latter with the selective marker GAP43).

No improvement was observed of pain scores, or PGP9.5 IENFs in skin biopsies collected at the site of the herniorrhaphy, following Capsaicin 8% Patch treatment of patients with chronic post-surgical groin pain (Bischoff et al., PLoS One. 2014; 9:e109144). In contrast, in patients with painful CIPN, a 30-minute application of Capsaicin 8% Patch to the feet and distal calf led to a significant reduction of pain scores, along with a significant increase of intra-epidermal and sub-epidermal nerve fibres. This was accompanied by an improvement in other mechanistic biomarkers i.e. epidermal levels of Nerve Growth Factor (NGF), Neurotrophin-3 (NT-3), and Langerhans cells (Anand et al., J Pain Res. 2019, 2:2039-2052).

### Example 2

### Extension to subjects suffering from non-painful diabetic peripheral neuropathy

The present investigation was extended to include subjects suffering from non-painful diabetic peripheral neuropathy. Patients with non-painful diabetic neuropathy are often prone to develop foot ulcers. Results are shown in Figures 19 to 28.

### Material and methods

### Study design

The study design for Example 2 was analogous to that of Example 1.

### Participants - subjects suffering from non-painful diabetic peripheral neuropathy

Patient demographics and characteristics are recorded in Table 4.

**Table 4**

| **Group** | **Non-painful Diabetic Neuropathy Patients** |
|---|---|
| Number of patients | n=17 |

| **Age, Years** | |
|---|---|
| Mean ± SD | 59 ± 13 |
| Range | 34-82 |

| **Sex, n (%)** | |
|---|---|
| Male | 14 (82%) |
| Female | 3 (18%) |

| **Type of Diabetes, n (%)** | |
|---|---|
| Type I | 13 (76%) |
| Type II | 4 (24%) |

| **Duration of Diabetes, Mean, Years (Range; SD)** | |
|---|---|
| | 30 (5-58; 16) |

| **Medications for Diabetes, n (%)** | |
|---|---|
| Insulin alone | 9 (53%) |
| Metformin alone | 1 (6%) |
| Insulin + Metformin | 4 (18%) |
| Insulin or Metformin + thiazolidinediones / gliptins / (SGLT2) inhibitors / sulfonylureas / GLP agonist | 4 (24%) |

| **HbA1c % mean (Range), mmol/mol mean (Range)** | |
|---|---|
| | 8 % (6.0 -9.0) |
| | 61 mmol/mol (41-74) |

| **Time elapsed since diagnosis of DPN, Mean; Years (Range; SD)** | |
|---|---|
| | 7 (3-15; 4) |

| **NCS (mean ± SEM; Range)** | |
|---|---|
| Peroneal motor action potential amplitude | 1.5 ± 1.4 (0.0-12.7) µV |
| Peroneal conduction velocity | 6.4 ± 6.4 (0.0-51.7) m/s |
| Sural sensory action potential amplitude | 3.2 ± 2.1 (0.0-17.0) µV |
| Sural nerve conduction velocity | 11.6 ± 7.6 (0.0-54.5) m/s |

| | |
|---|---|
| ***Abbreviations**:* SD, Standard Deviation; SGTL2, Sodium-glucose Cotransporter-2; GLP, Glucagon-like peptide; DPN, Diabetic Peripheral Neuropathy. | |

### Results

Table 5 shows patient assessments at baseline and 3 months follow-up (FU) after topical application of Capsaicin 8% patch.

**Table 5**

| **Quantitative sensory testing** | | | | |
|---|---|---|---|---|
| **PGIC** (Patient Global Impression of Change) | | | | |
| | | **Mean** | **SEM** | **p value (n=17)** |
| | Baseline | 4.0 | 0.0 | ** 0.009 |
| | 3 Months FU | 3.4 | 0.2 | |

| **Monofilament Thresholds** | | | | |
|---|---|---|---|---|
| Right calf | | **Mean** | **SEM** | **p value (n=17)** |
| | Baseline | 7.0 | 1.3 | 0.63 ns |
| | 3 Months FU | 7.4 | 1.3 | |

| Left Calf | | **Mean** | **SEM** | **p value (n=17)** |
|---|---|---|---|---|
| | Baseline | 6.8 | 1.1 | 0.05 ns |
| | 3 Months FU | 7.8 | 1.3 | |
| **Vibration Threshold** | | | | |

| Right Toe | | **Mean** | **SEM** | **p value (n=15)** |
|---|---|---|---|---|
| | Baseline | 30.2 | 3.9 | 0.22 ns |
| | 3 Months FU | 29.7 | 3.4 | |

| Left Toe | | **Mean** | **SEM** | **p value** (n=15) |
|---|---|---|---|---|
| | Baseline | 32.0 | 4.1 | 0.2 ns |
| | 3 Months FU | 28.9 | 3.4 | |
| **Thermal Thresholds** | | | | |

| **Cool Threshold** | | **Mean** | **SEM** | **p value (n=17)** |
|---|---|---|---|---|
| Right calf | Baseline | 11.9 | 2.6 | 0.59 ns |
| | 3 Months FU | 10.5 | 27 | |

| | | **Mean** | **SEM** | **p value (n=17)** |
|---|---|---|---|---|
| Left Calf | Baseline | 16.3 | 2.9 | 0.2 ns |
| | 3 Months FU | 12.4 | 2.5 | |

| **Warm threshold** | | **Mean** | **SEM** | **p value (n=17)** |
|---|---|---|---|---|
| Right calf | Baseline | 9.5 | 1.2 | 0.3 ns |
| | 3 Months FU | 8.6 | 1.2 | |

| | | **Mean** | **SEM** | **p value (n=17)** |
|---|---|---|---|---|
| Left Calf | Baseline | 12.3 | 0.9 | 0.6 ns |
| | 3 Months FU | 11.7 | 1.0 | |

| **Cold Pain Threshold** | | **Mean** | **SEM** | **p value (n=17)** |
|---|---|---|---|---|
| Right calf | Baseline | 21.4 | 2.6 | 0.7 ns |
| | 3 Months FU | 20.2 | 2.6 | |

| | | **Mean** | **SEM** | **p value (n=17)** |
|---|---|---|---|---|
| Left Calf | Baseline | 24.1 | 2.7 | 0.8 ns |
| | 3 Months FU | 24.9 | 2.4 | |

| **Heat Pain Threshold** | | **Mean** | **SEM** | **p value (n=17)** |
|---|---|---|---|---|
| Right calf | Baseline | 12.1 | 0.8 | 0.3 ns |
| | 3 Months FU | 13.1 | 0.9 | |

| | | **Mean** | **SEM** | **p value (n=17)** |
|---|---|---|---|---|
| Left Calf | Baseline | 14.4 | 0.6 | 0.1 ns |
| | 3 Months FU | 12.9 | 1.1 | |

| **Axon-reflex vasodilation (flux rise)** | | **Mean** | **SEM** | **p value (n=6)** |
|---|---|---|---|---|
| Right calf | Baseline | 13.6 | 3.8 | *0.035 |
| | 3 Months FU | 37.8 | 5.4 | |

When patients with non-painful diabetic peripheral neuropathy were treated with Capsaicin 8% patch (Qutenza) improved skin axon-reflex vasodilation (Figure 19) and nerve regeneration and restoration (Figures 20 to 26) were observed. Immunohistochemistry in skin biopsies for sub-epidermal vWF (blood vessel) density was also probed, with results as shown in Figures 27 and 28.

It is of note that axon-reflex vasodilation in the skin of patients suffering from non-painful diabetic peripheral neuropathy was significantly increased between baseline and 3 months follow-up visit (n=6, p=0.0355, paired t-test; see Figure 19). Previous studies of capsaicin-induced axon-reflex vasodilation showed that baseline values in the calf of similar patients with diabetic neuropathy were approximately 40% lower than in control subjects (Anand et al., Nat. Med. 1996, 2:703-707) suggesting that the increased flux after 3 months in the present study was within the normal range.

### Further discussion and conclusions

Improvement in sensory function in patients with DPN is important. In DPN dysfunction or loss of nociceptor fibres is associated with loss of protective sensation, regulation of local blood flow, and trophic changes, which are the major factors resulting in foot ulcers and amputations (Valabhji J, The Diabetic Foot Journal. 2011, 14(2):63-70, Armstrong and Boulton, N Engl J Med. 2017, 376(24):2367-2375). These complications are related to a higher risk of mortality (Brownrigg et al., J Vasc Surg. 2014, 60(4):982-6.e3). Volmer-Thole et al (Intl J Mol Sci. 2016, 17(6)) reported that among all diabetics the lifetime risk for developing diabetic foot ulceration is 25%, of which the majority will need amputation within four years of initial diagnosis. According to their epidemiological data, solely neuropathy is accountable for about 50% of the cases of diabetic foot syndrome, peripheral arterial occlusive disease (PAOD) on its own is accountable for just 15% of the cases, whereas in 35% a combination of both neuropathy and angiopathy play a role. We have previously reported an increase of sub-epidermal blood vessels with vWF in a different cohort of patients with diabetic peripheral neuropathy (Shillo et al., Diabet. Med. 2017; 34(S1):64), considered to be secondary to hypoxia and ischemia; the blood vessels density was unchanged after Capsaicin 8% Patch (Qutenza) treatment.

Overall, the present investigation shows that capsaicin reverses the natural decline of nerve fibre density, and warm sensory perception, in diabetic peripheral neuropathy, particularly at high doses such as that of the Capsaicin 8% Patch (Qutenza). It also appears to reverse the decline of the vascular response to stimulants, such as skin surface pressure, chemicals, and/or heat; and to reverse trophic changes that affect the maintenance of healthy skin tissue.

Administering capsaicin (particularly high doses, for example, Capsaicin 8% Patch) leads to considerable disease modification in patients suffering from DPN (whether painful or non-painful) involving the regeneration and restoration of cutaneous nerve fibre density, for both intra-epidermal (IENFs) and sub-epidermal nerve fibres (SENFs). Improving sensory function in patients with diabetic peripheral neuropathy in this way prevents the formation of diabetic foot ulcers in patients suffering from diabetic peripheral neuropathy. Regenerated peripheral nerves help to improve protective sensation, regulate the trophic response required to maintain healthy skin tissue (as well as regulate the trophic response required to restore healthy skin tissue following damage) and improve regulation of blood flow to areas of tissue subjected to pressure, so that adequate tissue perfusion is maintained and ulcers do not form. That is, particularly, the re-establishment of a more normal circulatory response, along with a more normal trophic response so as to maintain healthy skin tissue (and the trophic response required to restore healthy skin tissue following damage) helps prevent ulcer formation.

To summarise, the present data support the use of capsaicin, especially capsaicin at higher doses, to prevent foot ulcer recurrence, and ultimately the diabetic foot syndrome, in not only painful, but also non-painful diabetic peripheral neuropathy. Therefore, the present findings provide an exciting new prospect for the prevention of diabetic ulcers, e.g. diabetic foot ulcers, which are an important and increasing complication globally and can necessitate amputation of patients' ulcer-bearing limbs.

Where in the foregoing description, integers or elements are mentioned which have known, obvious or foreseeable equivalents, then such equivalents are herein incorporated as if individually set forth. Reference should be made to the claims for determining the true scope of the present invention, which should be construed so as to encompass any such equivalents. It will also be appreciated by the reader that integers or features of the invention that are described as preferable, advantageous, convenient or the like are optional and do not limit the scope of the independent claims. Moreover, it is to be understood that such optional integers or features, whilst of possible benefit in some embodiments of the invention, may not be desirable, and may therefore be absent, in other embodiments.

## Claims

1. Capsaicin, dihydrocapsaicin, nordihydrocapsaicin, homocapsaicin, homodihydrocapsaicin, capsiate, nonivamide or a TRPV1 agonist for use in the prevention of diabetic ulcers in a patient suffering from diabetic peripheral neuropathy, wherein the capsaicin, dihydrocapsaicin, nordihydrocapsaicin, homocapsaicin, homodihydrocapsaicin, capsiate, nonivamide or TRPV1 agonist is administered topically to one or more areas of the skin.

2. Capsaicin, dihydrocapsaicin, nordihydrocapsaicin, homocapsaicin, homodihydrocapsaicin, capsiate, nonivamide or a TRPV1 agonist for use according to claim 1, wherein the prevention of diabetic ulcers is achieved through the regeneration and restoration of sensory nerve fibres.

3. Capsaicin, dihydrocapsaicin, nordihydrocapsaicin, homocapsaicin, homodihydrocapsaicin, capsiate, nonivamide or a TRPV1 agonist for use according to claim 2, wherein the prevention of diabetic ulcers is mediated through one or more of: the full or partial restoration of an appropriate vascular response to skin surface pressure, chemical stimulants, and/or heat; the full or partial restoration of the trophic response required to maintain healthy skin tissue; and the full or partial restoration of protective sensation.

4. Capsaicin, dihydrocapsaicin, nordihydrocapsaicin, homocapsaicin, homodihydrocapsaicin, capsiate, nonivamide or a TRPV1 agonist for use according to claim 3, wherein the full or partial restoration of an appropriate vascular response to skin surface pressure, chemical stimulants, and/or heat comprises a full or partial restoration of the ability of the skin vasculature to dilate in response to the application of skin surface pressure, chemical stimulants, and/or heat.

5. Capsaicin, dihydrocapsaicin, nordihydrocapsaicin, homocapsaicin, homodihydrocapsaicin, capsiate, nonivamide or a TRPV1 agonist for use according to claim 3 or claim 4, wherein the full or partial restoration of the trophic response required to maintain healthy skin tissue comprises the full or partial restoration of an appropriate level of one or more growth factors in the tissue of the skin.

6. Capsaicin, dihydrocapsaicin, nordihydrocapsaicin, homocapsaicin, homodihydrocapsaicin, capsiate, nonivamide or a TRPV1 agonist for use according to any one of claims 1 to 5, wherein the capsaicin, dihydrocapsaicin, nordihydrocapsaicin, homocapsaicin, homodihydrocapsaicin, capsiate, nonivamide or TRPV1 agonist is capsaicin.

7. Capsaicin for use according to claim 6, wherein about 350 to about 1000 µg of capsaicin per cm² of skin (for example, about 500 to about 700 µg of capsaicin per cm² of skin) is administered to the one or more areas of the skin of the patient, particularly the distal leg and feet.

8. Capsaicin for use according to claim 6 or claim 7, wherein the capsaicin is administered in a cutaneous patch.

9. Capsaicin for use according to any one of claims 6 to 8, wherein administering capsaicin topically comprises administering Capsaicin 8% Patch to the one or more areas of the skin of the patient.

10. Capsaicin for use according to any one of claims 6 to 9, wherein capsaicin is administered topically to one or more areas of the skin of the patient for a period of about 30 minutes to about 60 minutes.

11. Capsaicin for use according to any one of claims 6 to 10, wherein the diabetic peripheral neuropathy includes painful diabetic peripheral neuropathy and/or non-painful diabetic peripheral neuropathy.

12. Capsaicin for use according to any one of claims 6 to 11, wherein the patient has previously had one or more diabetic ulcers.

13. Capsaicin for use according to any one of claims 6 to 12, wherein the diabetic ulcers include one or more diabetic foot ulcers.

## Patentansprüche

1. Capsaicin, Dihydrocapsaicin, Nordihydrocapsaicin, Homocapsaicin, Homodihydrocapsaicin, Capsiat, Nonivamid oder ein TRPV1-Agonist zur Verwendung bei der Vorbeugung von diabetischen Geschwüren bei einem Patienten, der an diabetischer peripherer Neuropathie leidet, wobei das Capsaicin, Dihydrocapsaicin, Nordihydrocapsaicin, Homocapsaicin, Homodihydrocapsaicin, Capsiat, Nonivamid oder der TRPV1 -Agonist topisch auf einen oder mehrere Hautbereiche appliziert wird.

2. Capsaicin, Dihydrocapsaicin, Nordihydrocapsaicin, Homocapsaicin, Homodihydrocapsaicin, Capsiat, Nonivamid oder ein TRPV1-Agonist zur Verwendung gemäß Anspruch 1, wobei die Prävention diabetischer Geschwüre durch die Regeneration und Wiederherstellung sensorischer Nervenfasern erreicht wird.

3. Capsaicin, Dihydrocapsaicin, Nordihydrocapsaicin, Homocapsaicin, Homodihydrocapsaicin, Capsiat, Nonivamid oder ein TRPV1-Agonist zur Verwendung gemäß Anspruch 2, wobei die Vorbeugung von diabetischen Geschwüren durch eines oder mehrere der folgenden Mittel vermittelt wird: die vollständige oder teilweise Wiederherstellung einer angemessenen vaskulären Reaktion auf Druck auf die Hautoberfläche, chemische Reize und/oder Wärme; die vollständige oder teilweise Wiederherstellung der trophischen Reaktion, die zur Erhaltung von gesundem Hautgewebe erforderlich ist; und die vollständige oder teilweise Wiederherstellung der Schutzempfindung.

4. Capsaicin, Dihydrocapsaicin, Nordihydrocapsaicin, Homocapsaicin, Homodihydrocapsaicin, Capsiat, Nonivamid oder ein TRPV1-Agonist zur Verwendung gemäß Anspruch 3, wobei die vollständige oder teilweise Wiederherstellung einer angemessenen vaskulären Reaktion auf Druck auf die Hautoberfläche, chemische Reize und/oder Wärme eine vollständige oder teilweise Wiederherstellung der Fähigkeit des Hautgefäßsystems umfasst, sich als Reaktion auf die Anwendung von Druck auf die Hautoberfläche, chemischen Reizen und/oder Wärme zu erweitern.

5. Capsaicin, Dihydrocapsaicin, Nordihydrocapsaicin, Homocapsaicin, Homodihydrocapsaicin, Capsiat, Nonivamid oder ein TRPV1-Agonist zur Verwendung gemäß Anspruch 3 oder Anspruch 4, wobei die vollständige oder teilweise Wiederherstellung der trophischen Reaktion, die zur Erhaltung von gesundem Hautgewebe erforderlich ist, die vollständige oder teilweisen Wiederherstellung eines angemessenen Spiegels eines oder mehrerer Wachstumsfaktoren im Hautgewebe.

6. Capsaicin, Dihydrocapsaicin, Nordihydrocapsaicin, Homocapsaicin, Homodihydrocapsaicin, Capsiat, Nonivamid oder ein TRPV1-Agonist zur Verwendung gemäß einem der Ansprüche 1 bis 5, wobei das Capsaicin, Dihydrocapsaicin, Nordihydrocapsaicin, Homocapsaicin, Homodihydrocapsaicin, Capsiat, Nonivamid oder der TRPV1-Agonist Capsaicin ist.

7. Capsaicin zur Verwendung gemäß Anspruch 6, wobei etwa 350 bis etwa 1000 µg Capsaicin pro cm² Haut (beispielsweise etwa 500 bis etwa 700 µg Capsaicin pro cm² Haut) auf einen oder mehrere Bereiche der Haut des Patienten, insbesondere auf den Unterschenkel und die Füße, aufgetragen werden.

8. Capsaicin zur Verwendung gemäß Anspruch 6 oder Anspruch 7, wobei das Capsaicin in einem Hautpflaster verabreicht wird.

9. Capsaicin zur Verwendung gemäß einem der Ansprüche 6 bis 8, wobei die topische Verabreichung von Capsaicin die Anwendung eines Capsaicin 8 %-Pflasters auf einen oder mehrere Bereiche der Haut des Patienten umfasst.

10. Capsaicin zur Verwendung gemäß einem der Ansprüche 6 bis 9, wobei Capsaicin über einen Zeitraum von etwa 30 Minuten bis etwa 60 Minuten topisch auf einen oder mehrere Bereiche der Haut des Patienten verabreicht wird.

11. Capsaicin zur Verwendung gemäß einem der Ansprüche 6 bis 10, wobei die diabetische periphere Neuropathie eine schmerzhafte diabetische periphere Neuropathie und/oder eine schmerzfreie diabetische periphere Neuropathie umfasst.

12. Capsaicin zur Verwendung gemäß einem der Ansprüche 6 bis 11, wobei der Patient zuvor ein oder mehrere diabetische Geschwüre hatte.

13. Capsaicin zur Verwendung gemäß einem der Ansprüche 6 bis 12, wobei die diabetischen Geschwüre ein oder mehrere diabetische Fußgeschwüre umfassen.

## Revendications

1. Capsaïcine, dihydrocapsaïcine, nordihydrocapsaïcine, homocapsaïcine, homodihydrocapsaïcine, capsiate, nonivamide ou un agoniste du TRPV1 pour son utilisation dans la prévention des ulcères diabétiques chez un patient souffrant de neuropathie périphérique diabétique, dans laquelle la capsaïcine, la dihydrocapsaïcine, la nordihydrocapsaïcine, l'homocapsaïcine, l'homodihydrocapsaïcine, le capsiate, le nonivamide ou l'agoniste du TRPV1 est administré(e) par voie topique sur une ou plusieurs zones de la peau.

2. Capsaïcine, dihydrocapsaïcine, nordihydrocapsaïcine, homocapsaïcine, homodihydrocapsaïcine, capsiate, nonivamide ou un agoniste du TRPV1 pour son utilisation selon la revendication 1, dans laquelle la prévention des ulcères diabétiques est obtenue par la régénération et la restauration des fibres nerveuses sensorielles.

3. Capsaïcine, dihydrocapsaïcine, nordihydrocapsaïcine, homocapsaïcine, homodihydrocapsaïcine, capsiate, nonivamide ou un agoniste du TRPV1 pour son utilisation selon la revendication 2, dans laquelle la prévention des ulcères diabétiques est médiée par l'un ou plusieurs de : la restauration complète ou partielle d'une réponse vasculaire appropriée à la pression superficielle cutanée, aux stimulants chimiques et/ou à la chaleur ; la restauration totale ou partielle de la réponse trophique nécessaire pour maintenir un tissu cutané sain ; et la restauration totale ou partielle de la sensation protectrice.

4. Capsaïcine, dihydrocapsaïcine, nordihydrocapsaïcine, homocapsaïcine, homodihydrocapsaïcine, capsiate, nonivamide ou un agoniste du TRPV1 pour son utilisation selon la revendication 3, dans laquelle la restauration complète ou partielle d'une réponse vasculaire appropriée à la pression superficielle cutanée, aux stimulants chimiques et/ou à la chaleur comprennent une restauration totale ou partielle de la capacité de la vascularisation cutanée à se dilater en réponse à l'application de pression superficielle cutanée, de stimulants chimiques et/ou de chaleur.

5. Capsaïcine, dihydrocapsaïcine, nordihydrocapsaïcine, homocapsaïcine, homodihydrocapsaïcine, capsiate, nonivamide ou un agoniste du TRPV1 pour son utilisation selon la revendication 3 ou la revendication 4, dans laquelle la restauration complète ou partielle de la réponse trophique nécessaire pour maintenir un tissu cutané sain comprend la restauration complète ou partielle d'un niveau approprié d'un ou de plusieurs facteurs de croissance dans le tissu de la peau.

6. Capsaïcine, dihydrocapsaïcine, nordihydrocapsaïcine, homocapsaïcine, homodihydrocapsaïcine, capsiate, nonivamide ou un agoniste du TRPV1 pour son utilisation selon l'une quelconque des revendications 1 à 5, dans laquelle la capsaïcine, la dihydrocapsaïcine, la nordihydrocapsaïcine, l'homocapsaïcine, l'homodihydrocapsaïcine, le capsiate, le nonivamide ou l'agoniste du TRPV1 est la capsaïcine.

7. Capsaïcine pour son utilisation selon la revendication 6, dans laquelle environ 350 à environ 1000 µg de capsaïcine par cm² de peau (par exemple, environ 500 à environ 700 µg de capsaïcine par cm² de peau) est administrée sur les une ou plusieurs zones de la peau du patient, en particulier la jambe et les pieds distaux.

8. Capsaïcine pour son utilisation selon la revendication 6 ou la revendication 7, dans laquelle la capsaïcine est administrée dans un patch cutané.

9. Capsaïcine pour son utilisation selon l'une quelconque des revendications 6 à 8, dans laquelle l'administration de capsaïcine par voie topique comprend l'administration d'un patch de capsaïcine à 8 % sur les une ou plusieurs zones de la peau du patient.

10. Capsaïcine pour son utilisation selon l'une quelconque des revendications 6 à 9, dans laquelle la capsaïcine est administrée par voie topique sur une ou plusieurs zones de la peau du patient pendant une période d'environ 30 minutes à environ 60 minutes.

11. Capsaïcine pour son utilisation selon l'une quelconque des revendications 6 à 10, dans laquelle la neuropathie périphérique diabétique inclut une neuropathie périphérique diabétique douloureuse et/ou une neuropathie périphérique diabétique non douloureuse.

12. Capsaïcine pour son utilisation selon l'une quelconque des revendications 6 à 11, dans laquelle le patient a précédemment présenté un ou plusieurs ulcères diabétiques.

13. Capsaïcine pour son utilisation selon l'une quelconque des revendications 6 à 12, dans laquelle les ulcères diabétiques incluent un ou plusieurs ulcères du pied diabétique.
